# EUROPEAN PATENT APPLICATION

(11) **EP 1 211 239 A1**
(43) Date of publication of application: **05.06.2002**
(21) Application number: 00956925.2
(22) Date of filing: 05.09.2000
(51) Int. Cl.: C07C 229/18, C07C 227/14, C07D 223/16

(54) **PROCESS FOR THE PREPARATION OF 2,3-DIHYDROAZEPINE COMPOUNDS**

(30) Priority: 06.09.1999 JP 25233499
(71) Applicant: Takeda Chemical Industries, Ltd., Osaka-shi, Osaka 541-0045 (JP)
(72) Inventor: IKEMOTO, Tomomi, Takarazuka-shi, Hyogo 665-0815 (JP); ITO, Tatsuya, 117.1-7 Asahigaoka-Kukakuseirichinai, Nara 639-0264 (JP); NISHIGUCHI, Atsuko, Itami-shi, Hyogo 664-0883 (JP); TOMIMATSU, Kiminori, Minoo-shi, Osaka 562-0034 (JP)
(74) Representative: Lewin, John Harvey
(86) International application number: JP0006012
(87) International publication number: WO0117947

(57) **Abstract**

As a process for preparing 2,3-dihydroazepine compounds at low cost in a simple and easy manner, there is provided a process for the preparation of compounds of the formula: 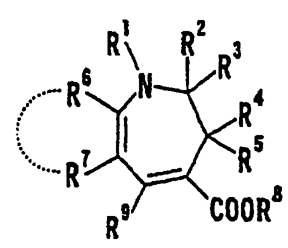 or salts thereof, characterized in that compounds of the formula: 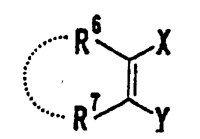 or salts thereof are reacted with compounds of the formula: 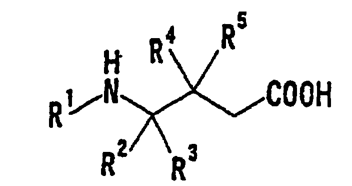 or salts thereof to give compounds of the formula: 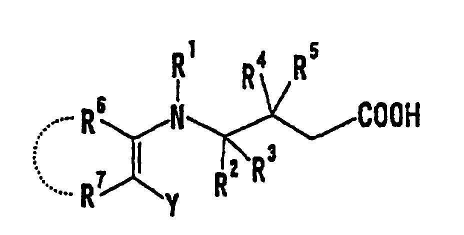 or salts thereof, which are then subjected to esterification and ring-closing reaction.

## Description

### Technical Field

The present invention relates to a process for the preparation of 2,3-dihydrobenzazepine compounds as useful intermediates for compounds having CCR5 antagonistic activity.

### Background Art

So far, for 2,3-dihydrobenzazpine derivatives, there has been described a process for synthesizing them by ring-closing diester derivatives of anthranilic acid by Dieckmann-type cyclization, followed by reduction and dehydration (U.S. Patent No. 4952573). However, this process has a number of steps and requires complicated procedures.

Under these circumstances, there has been desired a simple and easy process for the preparation of 2,3-dihydrobenzazepine derivatives.

### Disclosure of the Invention

The present inventors have extensively studied and, as a result, they have found a process for preparing 2,3-dihydrobenzazepine derivatives at low cost in a simple and easy manner using 4-(2-substituted-N-substituted anilino)-butyric acids as intermediates. Based on such knowledge, they have further studied and reached the completion of the present invention.

That is, the present invention relates to:
(1) a process for the preparation of a compound of the formula: 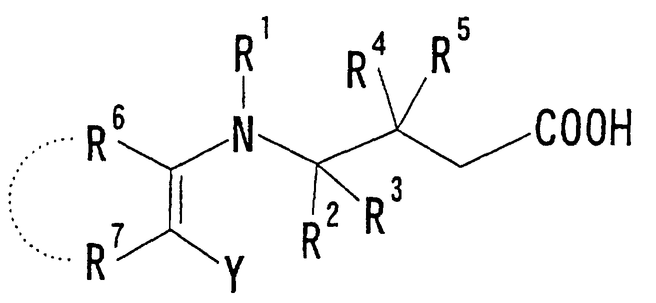 wherein each variable is as defined below, or a salt thereof, characterized in that a compound of the formula: 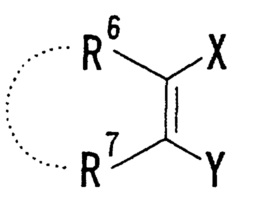 wherein X is a halogen atom; Y is an electron-withdrawing group; R⁶ and R⁷ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R⁶ and R⁷ may form a ring, or a salt thereof, is allowed to react with a compound of the formula: 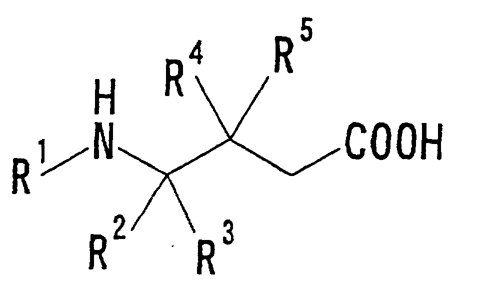 wherein R¹ is an optionally substituted hydrocarbon group, an optionally substituted acyl group, or an optionally substituted sulfonyl group; R², R³, R⁴, and R⁵ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R¹ and R², R¹ and R⁴, R² and R³, R⁴ and R⁵, or R² and R⁴ may form a ring, or a salt thereof;
(2) the preparation process as described above in (1), wherein Y is an optionally substituted acyl group;
(3) the preparation process as described above in (1), wherein R², R³, R⁴, and R⁵ are hydrogen atoms;
(4) the preparation process as described above in (1), wherein R¹ is an optionally substituted hydrocarbon group;
(5) a process for the preparation of a compound of the formula: 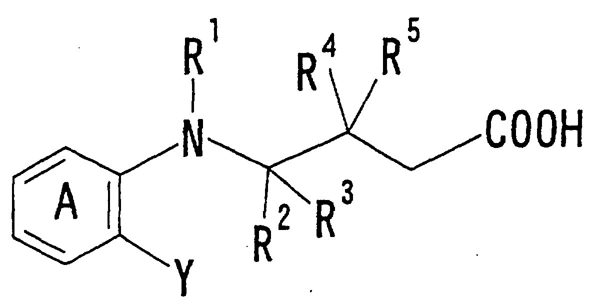 wherein each variable is as defined below, or a salt thereof, characterized in that a compound of the formula: 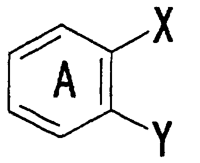 wherein X is a halogen atom; Y is an electron-withdrawing group; and ring A is an optionally substituted benzene ring, or a salt thereof, is allowed to react with a compound of the formula: 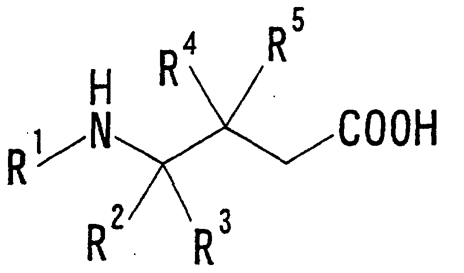 wherein R¹ is an optionally substituted hydrocarbon group, an optionally substituted acyl group, or an optionally substituted sulfonyl group; R², R³, R⁴, and R⁵ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R¹ and R², R¹ and R⁴, R² and R³, R⁴ and R⁵, or R² and R⁴ may form a ring, or a salt thereof;
(6) the preparation process as described above in (1), characterized in that a compound of the formula: 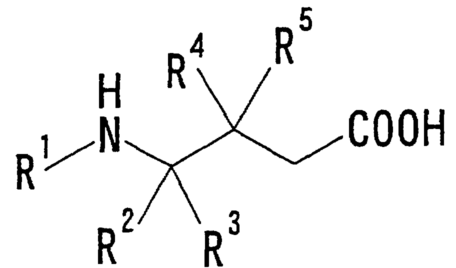 wherein each variable is as defined above in (1), or a salt thereof, is used, which is obtained by hydrolyzing a compound of the formula: 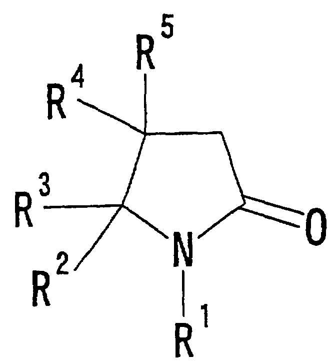 wherein each variable is as defined above in (1), or a salt thereof;
(7) the preparation process as described above in (6),
   characterized in that the compound of the formula: 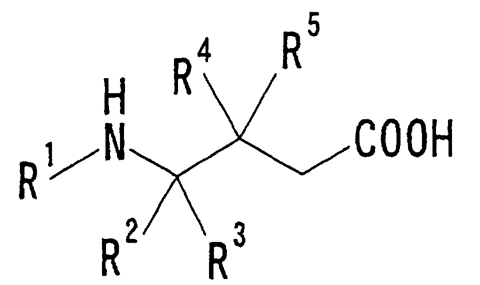 wherein each variable is as defined above in (1), or a salt thereof, is subjected, without being isolated, to the reaction with the compound of the formula: 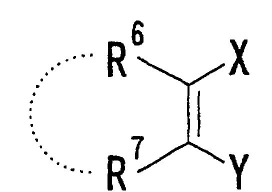 wherein each variable is as defined above in (1), or a salt thereof;
(8) a compound of the formula: 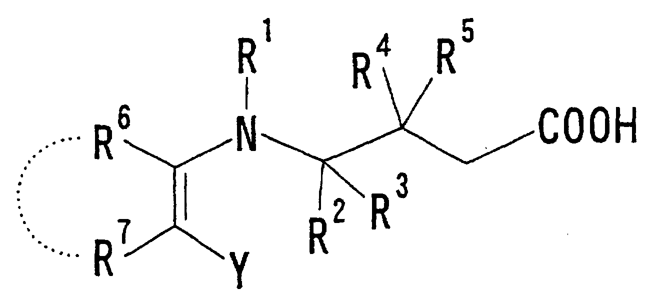 wherein Y is an electron-withdrawing group; R¹ is an optionally substituted hydrocarbon group, an optionally substituted acyl group, or an optionally substituted sulfonyl group; R², R³, R⁴, R⁵, R⁶, and R⁷ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R¹ and R², R¹ and R⁴, R² and R³, R⁴ and R⁵, R² and R⁴, or R⁶ and R⁷ may form a ring, or a salt thereof;
(9) the compound as described above in (8), wherein Y is an optionally substituted acyl group;
(10) the compound as described above in (8), wherein R², R³, R⁴, and R⁵ are hydrogen atoms;
(11) the compound as described above in (8), wherein R¹ is an optionally substituted hydrocarbon group;
(12) a compound of the formula: 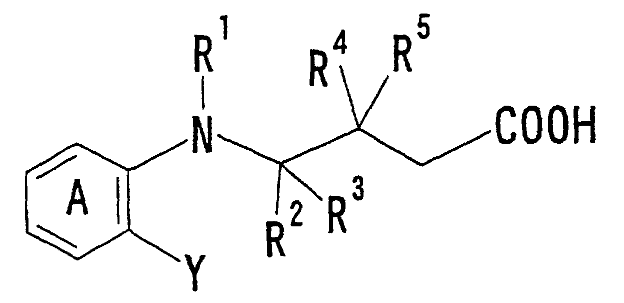 wherein Y is an electron-withdrawing group; ring A is an optionally substituted benzene ring; R¹ is an optionally substituted hydrocarbon group, an optionally substituted acyl group, or an optionally substituted sulfonyl group; R², R³, R⁴, and R⁵ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R¹ and R², R¹ and R⁴, R² and R³, R⁴ and R⁵, or R² and R⁴ may form a ring, or a salt thereof;
(13) the compound as described above in (12), wherein Y is an optionally substituted acyl group;
(14) the compound as described above in (12), wherein R², R³, R⁴, and R⁵ are hydrogen atoms;
(15) the compound as described above in (12), wherein R¹ is an optionally substituted hydrocarbon group;
(16) a process for the preparation of a compound of the formula: 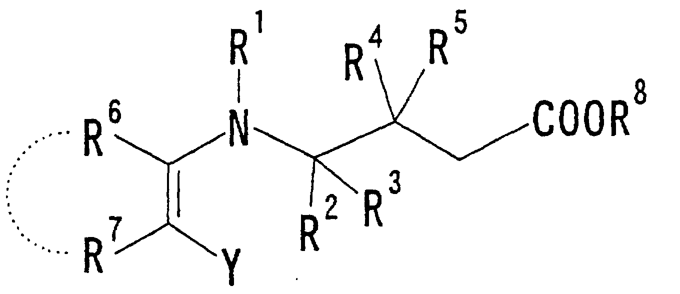 wherein R⁸ is an optionally substituted hydrocarbon group and the other variables are as defined below, or a salt thereof, characterized in that a compound of the formula: 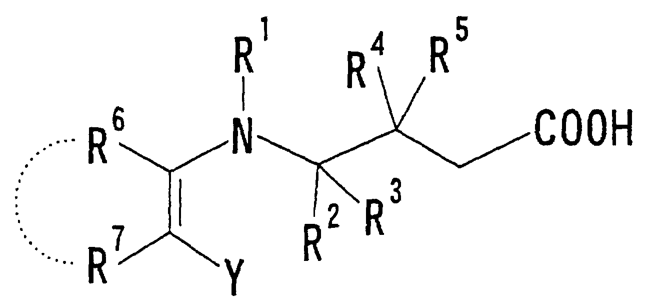 wherein each variable is as defined above in (1), or a salt thereof, which is obtained by the preparation process as described above in (1), is subjected to esterification;
(17) the preparation process as described above in (16), wherein Y is an optionally substituted acyl group;
(18) the preparation process as described above in (16), wherein R², R³, R⁴, and R⁵ are hydrogen atoms;
(19) the preparation process as described above in (16), wherein R¹ is an optionally substituted hydrocarbon group;
(20) a process for the preparation of a compound of the formula: 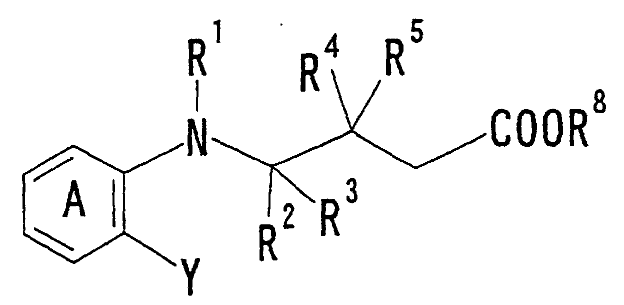 wherein R⁸ is an optionally substituted hydrocarbon group and the other variables are as defined below, or a salt thereof, characterized in that a compound of the formula: 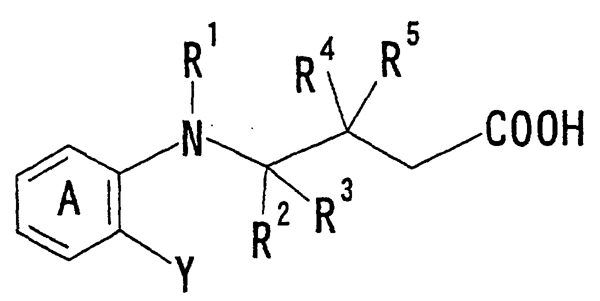 wherein each variable is as defined above in (5), or a salt thereof, which is obtained by the preparation process as described above in (5), is subjected to esterification;
(21) a process for the preparation of a compound of the formula: 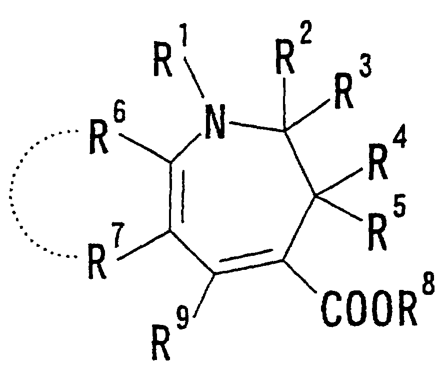 wherein R⁹ is a hydrogen atom or an optionally substituted hydrocarbon group, and the other variables are as defined below, or a salt thereof, characterized in that a compound of the formula: 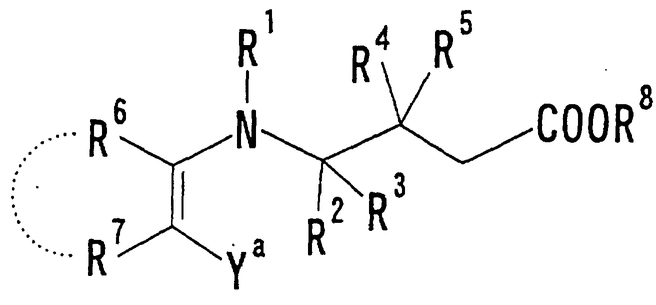 wherein Y^{a} is a group of formula -COR⁹ wherein R⁹ is a hydrogen atom or an optionally substituted hydrocarbon group, and the other variables are as defined above in (16), or a salt thereof, which is obtained by the preparation process as described above in (16), is subjected to ring-closing reaction;
(22) the preparation process as described above in (21), wherein R⁹ is a hydrogen atom;
(23) the preparation process as described above in (21), wherein R², R³, R⁴, and R⁵ are hydrogen atoms;
(24) the process as described above in (21), wherein R¹ is an optionally substituted hydrocarbon group;
(25) the preparation process as described above in (21), characterized in that a compound of the formula: 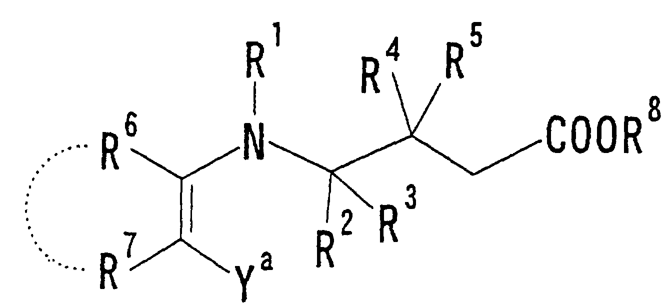 wherein Y^{a} is a group of formula -COR⁹ wherein R⁹ is a hydrogen atom or an optionally substituted hydrocarbon group, and the other variables are as defined above in (16), or a salt thereof, which is obtained by the preparation process as described above in (16), is subjected, without being isolated, to ring-closing reaction;
(26) a process for the preparation of a compound of the formula: 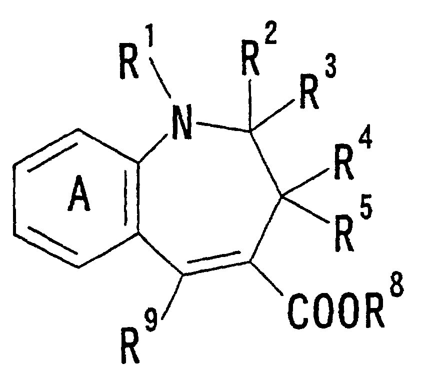 wherein R⁹ is a hydrogen atom or an optionally substituted hydrocarbon group, and the other variables are as defined below, or a salt thereof, characterized in that a compound of the formula: 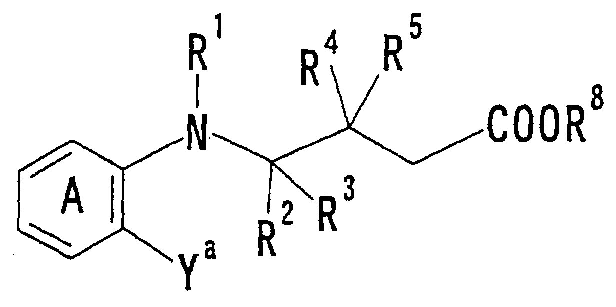 wherein Y^{a} is a group of formula -COR⁹ wherein R⁹ is a hydrogen atom or an optionally substituted hydrocarbon group, and the other variables are as defined above in (20), or a salt thereof, which is obtained by the preparation process as described above in (20), is subjected to ring-closing reaction;
(27) the preparation process as described above in (26), wherein R⁹ is a hydrogen atom;
(28) the preparation process as described above in (26), wherein R², R³, R⁴, and R⁵ are hydrogen atoms;
(29) the preparation process as described above in (26), wherein R¹ is an optionally substituted hydrocarbon group;
(30) the preparation process as described above in (26), characterized in that a compound of the formula: 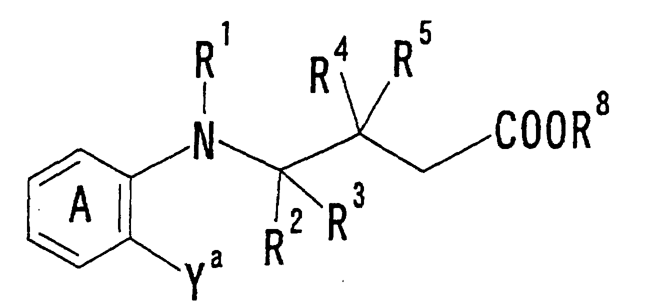
wherein Y^{a} is a group of formula -COR⁹ wherein R⁹ is a hydrogen atom or an optionally substituted hydrocarbon group, and the other variables are as defined above in (20), or a salt thereof, which is obtained by the preparation process as described above in (20), is subjected, without being isolated, to ring-closing reaction; and the like.

The "electron-withdrawing group" as used herein may include: (i) an optionally esterified or amidated carboxyl group; (ii) a group of formula -(CO)R²⁰ wherein R²⁰ is hydrogen or an optionally substituted hydrocarbon group; (iii) a nitrile group; (iv) a nitro group; (v) a group of formula -(SOₘ)R¹⁰ wherein m is 1 or 2, and R¹⁰ is an optionally substituted hydrocarbon group; (vi) a group of formula -PR¹¹R¹² wherein R¹¹ and R¹² are independently an optionally substituted hydrocarbon group; (vii) a group of formula -(PO) (OR¹³) (OR¹⁴) wherein R¹³ and R¹⁴ are independently hydrogen or an optionally substituted hydrocarbon group; (viii) an optionally substituted aryl group; (ix) an optionally substituted alkenyl group; (x) a halogen atom (*e.g.*, fluorine, chlorine, bromine, iodine); and (xi) a nitroso group. Preferred are an optionally esterified or amidated carboxyl group, a group of formula -(CO)R⁹, a nitrile group, a nitro group, a group of formula -(SOₘ)R¹⁰, a group of formula -PR¹¹R¹², and a group of formula (PO) (OR¹³) (OR¹⁴). More preferred are an optionally esterified carboxyl group (*e.g*., C₁₋₄ alkyl-esterified carboxyl group such as methoxycarbonyl, ethoxycarbonyl, and t-butoxycarbonyl).

The "esterified carboxyl group" in the "optionally esterified or amidated carboxyl group" described above in (i) may include a group of formula -(CO)OR¹⁵ wherein R¹⁵ is hydrogen or an optionally substituted hydrocarbon group. The "amidated carboxyl group" may include a group of formula -(CO)NR¹⁶R¹⁷ wherein R¹⁶ and R¹⁷ are independently hydrogen or an optionally substituted hydrocarbon group, or R¹⁶ and R¹⁷ may be combined together with an adjacent nitrogen atom to form 5- to 7-membered (preferably, 5- to 6-membered) cyclic amino (*e.g.*, tetrahydropyrrole, piperazine, piperidine, morpholine, thiomorpholine, pyrrole, imidazole).

In addition, in the formula described above in (vi) or (vii), R¹¹ and R¹² or R¹³ and R¹⁴ may be combined together to form lower (C₂₋₆) alkylene (*e.g.*, dimethylene, trimethylene, tetramethylene), lower (C₂₋₆) alkenylene (*e.g.*, -CH₂-CH=CH-, -CH₂-CH₂-CH=CH-, -CH₂-CH=CH-CH₂-), and lower (C₄₋₆) alkadienylene (*e.g*., -CH=CH-CH=CH-), preferably lower (C₁₋₆) alkylene, and more preferably lower (C₂₋₄) alkylene. These divalent groups may have a substituent(s). The substituent may include a hydroxyl group, halogen, C₁₋₄ alkyl, and C₁₋₄ alkoxy.

The "aryl group" in the optionally substituted aryl group described above in (viii) may include C₆₋₁₄ aryl such as phenyl and naphthyl, preferably C₆₋₁₀ aryl, and more preferably phenyl. The aryl group may have 1 to 3 substituents which are the same substituents as those optionally carried by the "optionally substituted hydrocarbon group" as described below.

The "alkenyl group" in the optionally substituted alkenyl group described above in (ix) may include alkenyl of 2 to 10 carbon atoms, such as vinyl, allyl, crotyl, 2-pentenyl, and 3-hexenyl, preferably lower (C₂₋₆) alkenyl, and more preferably vinyl. The alkenyl group may have 1 to 3 substituents which are the same substituents as those optionally carried by the "optionally substituted hydrocarbon group" as described below.

The "hydrocarbon group" in the "optionally substituted hydrocarbon group" as used herein may include:
(1) alkyl (*e.g*., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, and decyl, preferably lower (C₁₋₆) alkyl);
(2) cycloalkyl (*e.g.*, C₃₋₇ cycloalkyl such as cyclopropyl, cyclobutyl, cyclopentyl, cyclohexyl, and cycloheptyl);
(3) alkenyl (*e.g.*, alkenyl of 2 to 10 carbon atoms, such as vinyl, allyl, crotyl, 2-pentenyl, and 3-hexenyl, preferably lower (C₂₋₆) alkenyl);
(4) cycloalkenyl (*e.g*., cycloalkenyl of 3 to 7 carbon atoms, such as 2-cyclopentenyl, 2-cyclohexenyl, 2-cyclopentenylmethyl, and 2-cyclohexenylmethyl);
(5) alkynyl (*e.g.*, alkynyl of 2 to 10 carbon atoms, such as ethynyl, 1-propynyl, 2-propynyl, 1-butynyl, 2-pentynyl, and 3-hexynyl, preferably lower (C₂₋₆) alkynyl);
(6) aryl (*e.g.*, C₆₋₁₄ aryl such as phenyl and naphthyl, preferably C₆₋₁₀ aryl, and more preferably phenyl);
(7) aralkyl (*e.g.,* phenyl-C₁₋₄ alkyl (*e.g.,* benzyl and phenethyl)); and the like.

Among all these groups, preferred is alkyl, more preferred is C₁₋₄ alkyl such as methyl or ethyl, and in particular, preferably used is methyl.

The hydrocarbon group may have a substituent(s). The substituent may include halogen (*e.g.*, fluorine, chlorine, bromine, iodine), nitro, cyano, a hydroxyl group, an optionally substituted thiol group (*e.g.*, thiol, C₁₋₄ alkylthio), an optionally substituted amino group (*e.g.*, amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, and imidazole), an optionally esterified or amidated carboxyl group *(e.g.,* carboxyl, C₁₋₄ alkoxy carbonyl, carbamoyl, mono-C₁₋₄ alkylcarbamoyl, di-C₁₋₄ alkylcarbamoyl), C₁₋₄ alkyl optionally substituted with a halogen atom or C₁₋₄ alkoxy (*e.g.*, trifluoromethyl, methyl, ethyl), C₁₋₄ alkoxy optionally substituted with a halogen atom or C₁₋₄ alkoxy (e.g., methoxy, ethoxy, trifluoromethoxy, and trifluoroethoxy), formyl, C₂₋₄ alkanoyl (*e.g.*, acetyl, propionyl), C₁₋₄ alkylsulfonyl (*e.g.*, methanesulfonyl, ethanesulfonyl), and C₁₋₄ alkylsulfinyl (*e.g.*, methanesulfinyl, ethanesulfinyl). The number of substituents may preferably be 1 to 3.

In the above formulas, R¹ and R², R¹ and R⁴, R² and R³, R⁴ and R⁵, or R² and R⁴ may be combined together with an adjacent carbon atom or with adjacent carbon and nitrogen atoms to form a ring by forming lower (C₂₋₆) alkylene (*e.g.,* dimethylene, trimethylene, tetramethylene), lower (C₂₋₆) alkenylene (*e.g.*, -CH₂-CH=CH-, -CH₂-CH₂-CH-CH-, -CH2-CH=CH-CH₂), and lower (C₄₋₆) alkadienylene (*e.g*., -CH=CH-CH=CH-), preferably lower (C₁₋₆) alkylene, and more preferably lower (C₂₋₄) alkylene. In addition, these divalent groups may have a substituent(s). The substituent may include a hydroxyl group, halogen, C₁₋₄ alkyl, and C₁₋₄ alkoxy.

The "halogen atom" represented by X, R², R³, R⁴, R⁵, R⁶, or R⁷ in the above formulas may include fluorine, chlorine, bromine, and iodine.

The "optionally substituted amino group" represented by R², R³, R⁴, R⁵, R⁶, or R⁷ in the above formulas may include the amino group optionally substituted with the "optionally substituted hydrocarbon group" as described above. The number of substituents may be any of 0 to 2 and, when two substituents are present, these two substituents may be the same or different. Alternatively, two substituents may be combined together with an adjacent nitrogen atom to form 5- to 7-membered (preferably, 5- to 6-membered) cyclic amino (*e.g*., tetrahydropyrrole, piperazine, piperidine, morpholine, thiomorpholine, pyrrole, imidazole).

The "optionally substituted hydroxyl group" represented by R², R³, R⁴, R⁵, R⁶, or R⁷ in the above formulas may include a hydroxyl group optionally substituted with the "optionally substituted hydrocarbon group" as described above.

The "optionally substituted thiol group" represented by R², R³, R⁴, R⁵, R⁶, or R⁷ in the above formulas may include a thiol group optionally substituted with the "optionally substituted hydrocarbon group" as described above.

The "heterocyclic ring" in the "optionally substituted heterocyclic group" represented by R², R³, R⁴, R⁵, R⁶, or R⁷ in the above formulas may include a 5- to 7-membered aromatic heterocyclic ring, and a saturated or unsaturated non-aromatic heterocyclic ring (aliphatic heterocyclic ring) containing at least one (preferably 1 to 4, more preferably 1 or 2) of one to three kinds (preferably, one to two kinds) of heteroatoms selected from an oxygen atom, a sulfur atom, a nitrogen atom, and the like.

The "aromatic heterocyclic ring" may include 5- to 6-membered aromatic monocyclic heterocyclic rings (*e.g.*, furan, thiophene, pyrrole, oxazole, isooxazole, thiazole, isothiazole, imidazole, pyrazole, 1,2,3-oxadiazole, 1,2,4-oxadiazole, 1,3,4-oxadiazole, furazane, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine). The "non-aromatic heterocyclic ring" may include 5- to 7-membered (preferably, 5- to 6-membered) saturated or unsaturated (preferably, saturated) non-aromatic heterocyclic rings (aliphatic heterocyclic rings) such as pyrrolidine, tetrahydrofuran, thiolane, piperidine, tetrahydropyran, morpholine, thiomorpholine, piperazine, and pyran); and 5- to 6-membered non-aromatic heterocyclic rings in which some or all of the double bonds in the above aromatic monocyclic heterocyclic rings have been saturated. As the heterocyclic rings, preferred are 5- to 6-membered aromatic rings. Further, furan, thiophene, pyrrole, and pyridine (preferably, 6-membered ring) are preferred.

The substituents optionally carried by the heterocyclic rings may include the same substituents as those optionally carried by the "optionally substituted hydrocarbon group" as described above. The number of substituents may be 1 to 3.

In the above formulas, the Y may preferably be an optionally substituted acyl group. In particular, preferred is a group of the formula -(CO)R²⁰ wherein R²⁰ is hydrogen or an optionally substituted hydrocarbon group. The R¹ may preferably be an optionally substituted hydrocarbon group. The R², R³, R⁴, or R⁵ may preferably be a hydrogen atom or an optionally substituted hydrocarbon group, more preferably a hydrogen atom. The R⁹ may preferably be a hydrogen atom. The R⁶ or R⁷ may preferably be combined together to form an optionally substituted benzene ring.

The substituents optionally carried by the "optionally substituted benzene ring" as used herein may include the same substituents as those optionally carried by the "optionally substituted hydrocarbon group" as described above; the above "optionally substituted aryl group" which may be bonded through a spacer (*e.g.*, a divalent group of 1 to 4 atoms forming a straight chain moiety) (preferably, the above "optionally substituted aryl group" which is directly bonded). In particular, it may preferably be an electron-donating group. The number of substituents may be 1 to 4.

The "spacer" may include -(CH₂)ₐ- [wherein a is an integer of 1 to 4 (preferably, integer of 1 to 2)], -(CH₂)_{b}-X'- [wherein b is an integer of 0 to 3 (preferably 0 or 1) and X' is an optionally substituted imino group (*e.g*., an imino group optionally substituted with lower (C₁₋₆) lower alkyl, lower (C₃₋₇) cycloalkyl, formyl, lower (C₂₋₇) lower alkanoyl, lower (C₁₋₆) lower alkoxy-carbonyl, or the like), a carbonyl group, an oxygen atom or an optionally oxidized sulfur atom (*e.g.,* -S(O)ₙ- wherein n is an integer of 0 to 2)], -CH=CH-, -C≡C-, -CO-NH-, and -SO₂-NH-(preferably-(CH₂)_{b}-X'-, more preferably -CH₂-O-). These groups may be bonded with the "optionally substituted benzene ring" through either their right or left bond. They may preferably be bonded with the "optionally substituted benzene ring" through their right bond.

The ring formed by combining R⁶ and R⁷ may include 5-to 7-membered (preferably, 5- to 6-membered) unsaturated alicyclic hydrocarbons such as C₅₋₇ cycloalkene *(e.g.,* 1-cyclopentene, 2-cyclopentene, 3-cyclopentene, 2-cyclohexne, 3-cyclohexne) and C₅₋₆ cycloalkadiene (*e.g.*, 2,4-cyclopentadiene, 2,4-cyclohexadiene, 2,4-cyclohexadiene); 6-membered aromatic hydrocarbons such as benzene; 5- to 7-membered aromatic heterocyclic rings and unsaturated non-aromatic heterocyclic rings (aliphatic heterocyclic rings), which contain at least one (preferably 1 to 4, more preferably 1 or 2) of one to three kinds (preferably, one or two kinds) of heteroatoms selected from an oxygen atom, a sulfur atom, a nitrogen atom, and the like.

The "aromatic heterocyclic rings" may include 5- to 6-membered aromatic monocyclic heterocyclic rings (*e.g.*, furan, thiophene, pyrrole, oxazole, isooxazole, thiazole, isothiazole, imidazole, pyrazole, 1,2,3-oxadiazole, 1,2,3-thiadiazole, 1,2,3-triazole, pyridine, pyridazine, pyrimidine, pyrazine). The "non-aromatic heterocyclic rings" may include 5- to 6-membered non-aromatic heterocyclic rings in which some of the double bonds in the above aromatic monocyclic heterocyclic rings are saturated.

The ring formed by combining R⁶ and R⁷ may preferably be a 5- to 6-membered aromatic ring. Further, preferred are benzene, furan, thiophene, pyrrole, and pyridine (preferably, 6-membered rings). In particular, benzene is preferred.

The ring formed by combining R⁶ and R⁷ may have a substituent(s). The substituents may include the same substituents as those optionally carried by the benzene ring in the "optionally substituted benzene ring" as described above. One to three of the same or different substituents may be attached to any replaceable position.

The leaving group used herein may include a halogen atom (*e.g.*, fluorine, chlorine, bromine, iodine) and a group of formula -O(SOₘ)R wherein m is 1 or 2, and R is an optionally substituted hydrocarbon group (preferably, optionally halogenated C₁₋₄ alkyl, and more preferably trifluoromethyl). Among all these groups, preferred is a halogen atom, and particularly preferred is a fluorine atom.

The "optionally substituted acyl group" represented by R¹ in the above formulas may include a group of formula -(CO)R' wherein R' is a hydrogen atom, an optionally substituted hydrocarbon group, or an optionally substituted heterocyclic group. The "optionally substituted heterocyclic group" represented by R' may include the same as the "optionally substituted heterocyclic group" represented by R², R³, R⁴, R⁵, R⁶, or R⁷.

The "substituted sulfonyl group" represented by R¹ in the above formulas may include a group of formula -(SO₂)R" wherein R'' is an optionally substituted hydrocarbon group or an optionally substituted heterocyclic group. The "optionally substituted heterocyclic group" represented by P'' may include the same as the "optionally substituted heterocyclic group" represented by R², R³, R⁴, R⁵, R⁶, or R⁷.

Depending upon the kinds of substituents as described above, when compounds having these substituents are basic compounds, they can be converted into salts by the use of an acid according to the ordinary method. The acid may be any one, so long as it does not adversely affect the reaction. Examples thereof may include inorganic acids such as hydrochloric acid, hydrobromic acid, phosphoric acid, sulfuric acid, nitric acid, and sulfamic acid; organic acids such as formic acid, acetic acid, trifluoroacetic acid, tartaric acid, citric acid, fumaric acid, maleic acid, succinic acid, malic acid, p-toluenesulfonic acid, methanesulfonic acid, and benzenesulfonic acid; and acidic amino acids such as aspartic acid and glutamic acid. When the resulting compounds are salts, they may be converted into free bases according to the ordinary method.

On the other hand, depending upon the kinds of substituents as described above, when compounds having these substituents are acidic compounds, they can be converted into salts by the use of a base according to the ordinary method. The salt with a base may be a salt with any base, so long as it does not adversely affect the reaction. Examples thereof may include salts with inorganic bases, salts with organic bases, and salts with basic amino acids. Preferred examples of the salts with inorganic bases may include alkali metal salts such as sodium salts and potassium salts; alkaline earth metal salts such as calcium salts and magnesium salts; as well as aluminum salts and ammonium salts. Preferred examples of the salts with organic bases may include salts with trimethylamine, triethylamine, pyridine, picoline, ethanolamine, diethanolamine, triethanolamine, dicyclohexylamine, and N,N'-dibenzylethylenediamine. Preferred examples of the salts with basic amino acids may include salts with arginine, lysine, and ornithine. When the resulting compounds are salts, they may be converted into free acids according to the ordinary method.

The reaction described above in (1) is carried out, for example, under the following reaction conditions.

A compound of the formula: 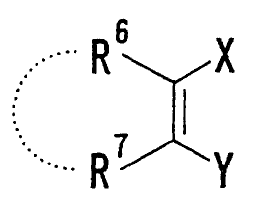 wherein each variable is as defined above, or a salt thereof, is reacted with a compound of the formula: 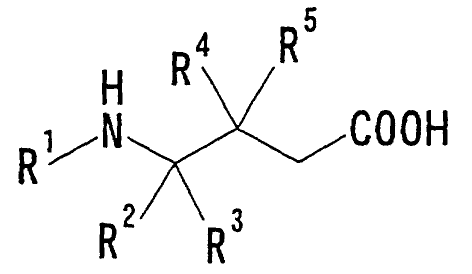 wherein each variable is as defined above, or a salt thereof, to give a compound of the formula: 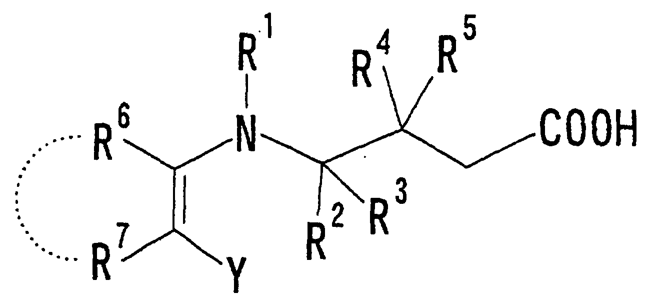 wherein each variable is as defined above, or a salt thereof.

The reaction described above in (1) is preferably carried out in the presence of a base. The base may include metal hydrides (*e.g*., alkali metal hydrides such as sodium hydride and potassium hydride), metal hydrocarbons (*e.g.*, compounds each having a chemical bond in which C₁₋₄ alkyl is directly bonded with an alkali metal, such as n-butyl lithium), alcoholates (*e.g.*, compounds in which the hydrogen of a hydroxyl group in a C₁₋₄ alcohol is replaced with an alkali metal, such as NaOMe, NaOEt, t-BuONa, and t-BuOK), alkali metal hydroxides (*e.g.*, NaOH, KOH), basic carbonates (*e.g.,* carbonates with alkali metal salts such as sodium salts, potassium salts, and cesium salts, or with alkaline earth metal salts such as calcium salts and magnesium salts), basic bicarbonates (*e.g.*, hydrogencarbonates with alkali metal salts such as sodium salts and potassium salts), organic bases (*e.g.*, trimethylamine, triethylamine, diisopropylethylamine, pyridine, picoline, N-methylpyrrolidine, N-methylmorpholine, 1,5-diazabicyclo[4.3.0]non-5-ene, 1,4-diazabicyclo[2.2.2]octane, and 1,8-diazabicyclo[5.4.0]-7-undecene). Among all these bases, preferably used are basic carbonates (*e.g.*, carbonates with alkali metal salts such as sodium salts, potassium salts, and cesium salts, or with alkaline earth metal salts such as calcium salts and magnesium salts).

The amount of base used in the reaction described above in (1) is about 0.1 to 100 equivalents, preferably about 0.5 to 5 equivalents.

Relative to the compound of the formula: 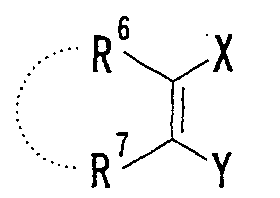 wherein each variable is as defined above, or a salt thereof, the compound of the formula: 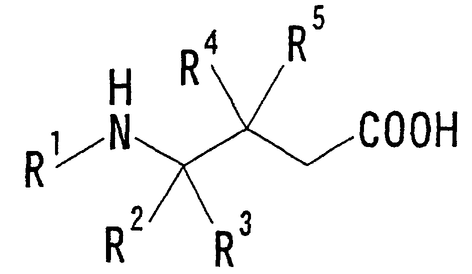 wherein each variable is as defined above, or a salt thereof, is used at about 0.1 to 100 equivalents, preferably about 0.5 to 5 equivalents.

As the reaction solvent, used are halogen solvents (*e.g.*, methylene chloride, dichloroethane, chloroform), aliphatic hydrocarbons (*e.g.*, n-hexane), aromatic hydrocarbons (*e.g*., benzene, toluene), ethers (*e.g*., tetrahydrofuran (THF), diethyl ether, isopropyl ether (IPE)), polar solvents (*e.g*., water, acetonitrile, dimethylformamide (DMF), dimethyl sulfoxide (DMSO), dimethylacetamide (DMAC)), and protonic solvents (*e.g.*, alcohols such as methanol, ethanol, propanol, isopropanol, n-butanol, and 2-methoxyethanol). In the reaction, a suitable mixed solvent may be used. Among all these solvents, preferably used are polar solvents (preferably, water-containing DMSO and water-containing DMAC).

The reaction temperature is usually about 0°C to 200°C, preferably about 50°C to 150°C. The reaction time is usually about 0.5 to 100 hours, preferably about 1 to 50 hours.

The reaction described above in (5) is carried out, for example, by the following step.

The compound of the formula: 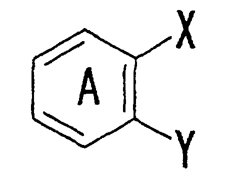 wherein each variable is as defined above, or a salt thereof, is reacted with the compound of the formula: 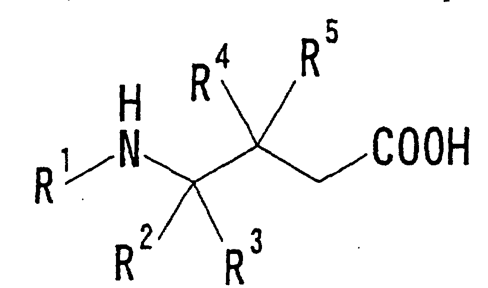 wherein each variable is as defined above, or a salt thereof, to give a compound of the formula: 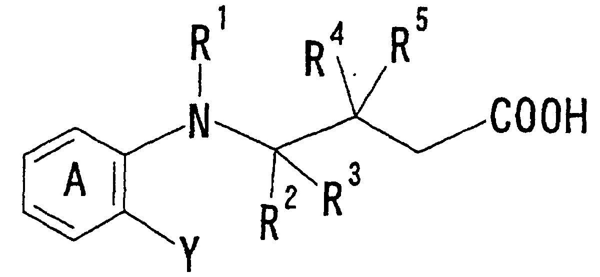 wherein each variable is as defined above, or a salt thereof.

The reaction described above in (5) is carried out under the same reaction conditions as those for the reaction described above in (1).

The reaction described above in (6) is carried out, for example, under the following reaction conditions:

The compound of the formula: 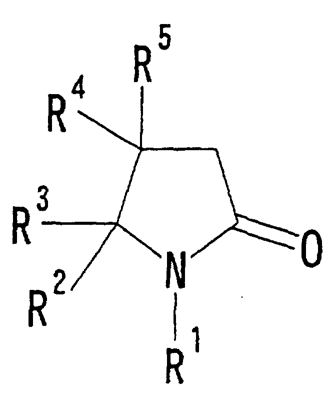 wherein each variable is as defined above, or a salt thereof, is hydrolyzed to give a compound of the formula: 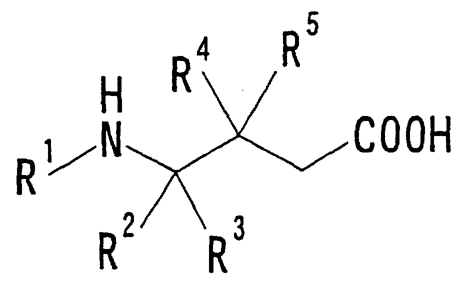 wherein each variable is as defined above, or a salt thereof, and the resulting compound or a salt thereof is subjected to the reaction described above in (1) to give a compound of the formula: 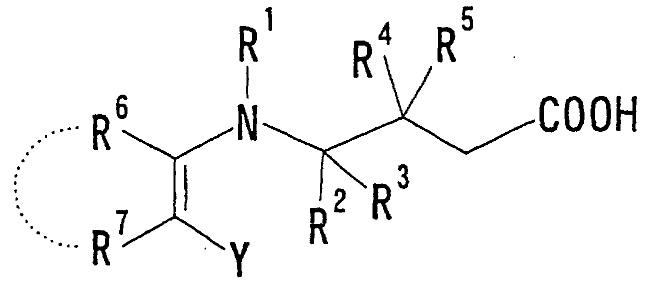 wherein each variable is as defined above, or a salt thereof. In this reaction, the intermediate may or may not be isolated; however, two steps may preferably be carried out continuously without isolating the intermediate.

The above hydrolysis can be carried out according to the method known per se and, for example, can be carried out by hydrolysis with an acid or an alkali. As the acid, for example, hydrochloric acid, sulfuric acid, or hydrobromic acid is used. As the alkali, lithium hydroxide, potassium hydroxide, sodium hydroxide, calcium hydroxide, or barium hydroxide is used.

The reaction described above in (16) is carried out, for example, under the following reaction conditions.

The compound of the formula: 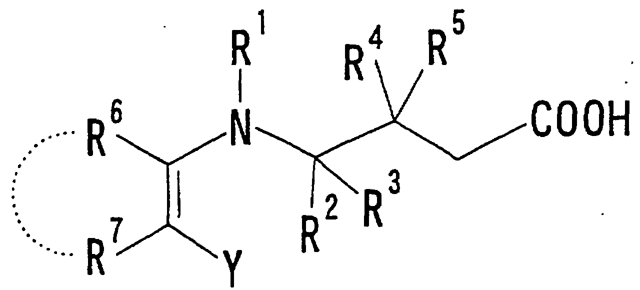 wherein each variable is as defined above, or a salt thereof, which is obtained by the reaction described above in (1), is subjected to esterification to give a compound of the formula: 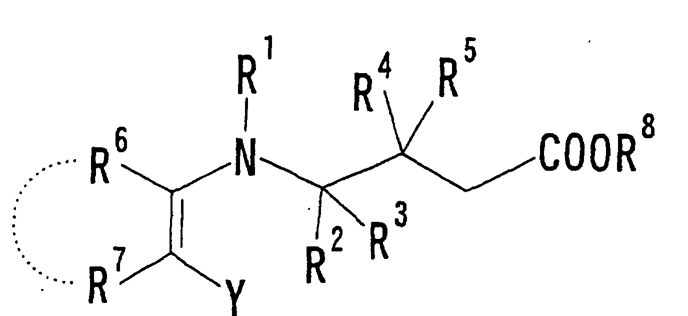 wherein each variable is as defined above, or a salt thereof.

The esterification is a reaction for converting free acids into esters by the means known per se. For example, esters can be prepared by reacting free acids or their reactive derivatives with alcohols (R⁸-OH) corresponding to substituent R⁸, or reacting free acids or their salts with halogenated alkyl compounds (X-R⁵ wherein X is a halogen atom) corresponding to substituent R⁸.

The reaction described above in (20) is carried out, for example, by the following step.

The compound of the formula: 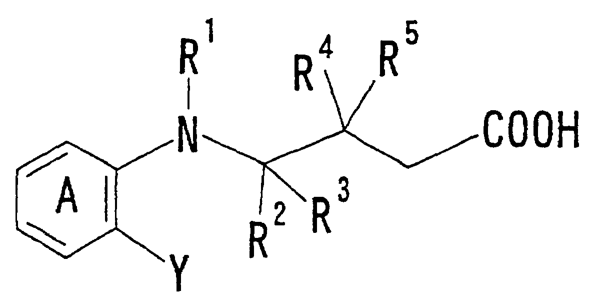 wherein each variable is as defined above, or a salt thereof, which is obtained by the process described above in (5), is subjected to esterification to give a compound of the formula: 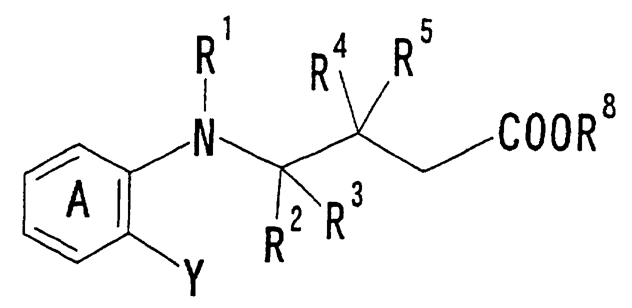 wherein each variable is as defined above, or a salt thereof.

The reaction described above in (20) is carried out, for example, under the same reaction conditions as those for the reaction described above in (16).

The reaction described above in (21) is carried out, for example, under the following conditions.

The compound of the formula: 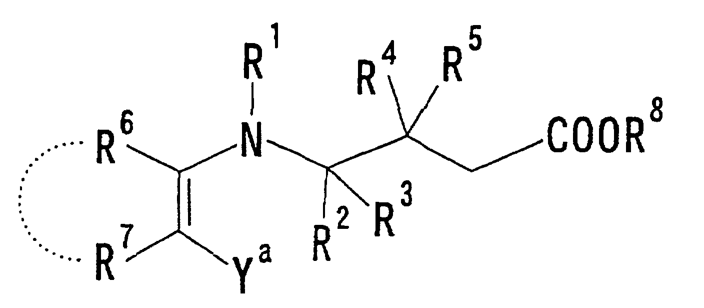 wherein each variable is as defined above, or a salt thereof, which is obtained by the reaction described above in (16), is subjected to ring-closing reaction to give a compound of the formula: 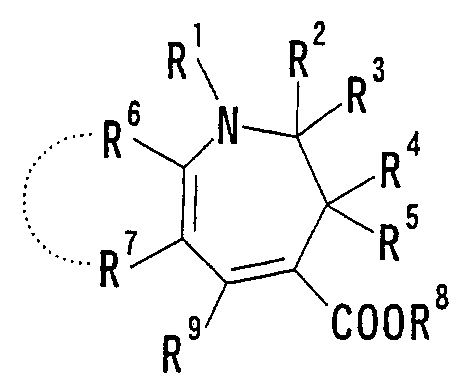 wherein each variable is as defined above, or a salt thereof. In this reaction, the intermediate obtained by the reaction described above in (16) may or may not be isolated; however, two steps may preferably be carried out continuously without isolating the intermediate.

The reaction described above in (21) may preferably be carried out in the presence of a base. The base may include metal hydrides (*e.g.,* alkali metal hydrides such as sodium hydride and potassium hydride), metal hydrocarbons (*e.g.,* compounds each having a chemical bond in which C₁₋₄ alkyl is directly bonded with an alkali metal, such as n-butyl lithium), alcoholates (*e.g.,* compounds in which the hydrogen of a hydroxy group in a C₁₋₄ alcohol is replaced with an alkali metal, such as NaOMe, NaOEt, t-BuONa, and t-BuOK), alkali metal hydroxides (*e.g.*, NaOH, KOH), basic carbonates (*e.g*., carbonates with alkali metal salts such as sodium salts, potassium salts, and cesium salts, or with alkaline earth metal salts such as calcium salts and magnesium salts), basic bicarbonates (*e.g*., bicarbonates with alkali metal salts such as sodium salts and potassium salts), organic bases (*e.g.*, trimethylamine, triethylamine, diisopropylethylamine, pyridine, picoline, N-methylpyrrolidine, N-methylmorpholine, 1,5-diazabicyclo[4.3.0]non-5-ene, 1,4-diazabicyclo[2.2.2]octane, 1,8-diazabicyclo[5.4.0]-7-undecene). Among all these bases, preferably used are alcoholates (*e.g.*, compounds in which the hydrogen of a hydroxy group in a C₁₋₄ alcohol is replaced with an alkali metal, such as NaOMe, NaOEt, t-BuONa, and t-BuOk).

The amount of base used in the reaction described above in (21) is about 0.1 to 100 equivalents, preferably about 0.5 to 5 equivalents.

As the reaction solvent, used are halogen solvents (*e.g*., methylene chloride, dichloroethane, chloroform), aliphatic hydrocarbons (*e.g.,* n-hexane), aromatic hydrocarbons (*e.g.,* benzene, toluene), ethers (*e.g.*, tetrahydrofuran (THF), diethyl ether, isopropyl ether (IPE)), polar solvents (*e.g.*, water, acetonitrile, dimethylformamide (DMF), dimethyl sulfoxide (DMSO), DMAC), protonic solvents (*e.g.*, alcohols such as methanol, ethanol, propanol, isopropanol, n-butanol, and 2-methoxyethanol), carbonic acid diesters (*e.g.*, carbonic acid di-C₁₋₄ alkyl esters such as dimethyl carbonate and diethyl carbonate), formic acid esters (*e.g.*, formic acid C₁₋₄ alkyl esters), and oxalic acid diesters (*e.g*., oxalic acid di-C₁₋₄ alkyl ester). In the reaction, a suitable mixed solvent may be used. Among all these solvents, preferably used are solvents containing carbonic acid diesters.

The reaction temperature is usually about -20°C to 200°C, preferably about 10°C to 100°C. The reaction time is usually about 0.1 to 100 hours, preferably about 0.5 to 50 hours.

The reaction described above in (26) is carried out, for example, by the following step.

The compound of the formula: 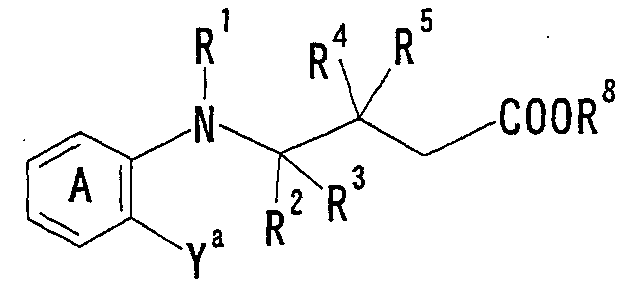 wherein each variable is as defined above, or a salt thereof, which is obtained by the reaction described above in (20), is subjected to ring-closing reaction to give a compound of the formula: 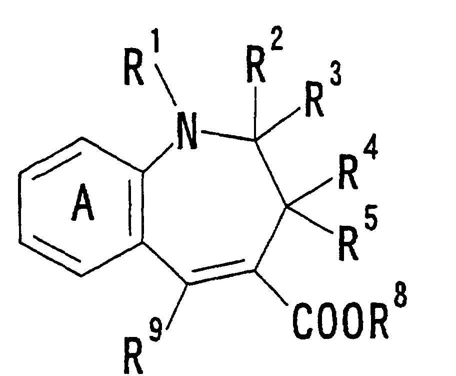 wherein each variable is as defined above, or a salt thereof.

The reaction described above in (26) is carried out, for example, under the same reaction conditions as those for the reaction described above in (21).

The compound of the formula: 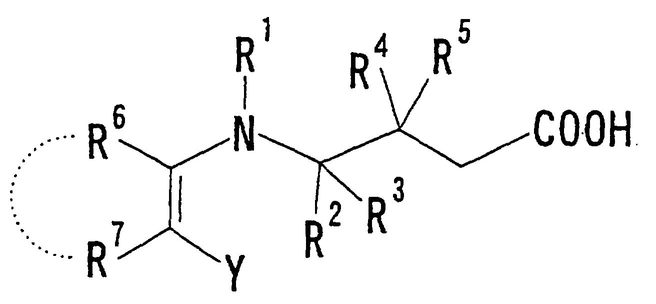 wherein Y is an electron-withdrawing group; R¹ is an optionally substituted hydrocarbon group, an optionally substituted acyl group, or a substituted sulfonyl group; R², R³, R⁴, R⁵, R⁶, and R⁷ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R¹ and R², R¹ and R⁴, R² and R³, R⁴ and R⁵, R² and R⁴, or R⁶ and R⁷ may form a ring, or a salt thereof, which is obtained by the reaction described above in (1); and the compound of the formula: 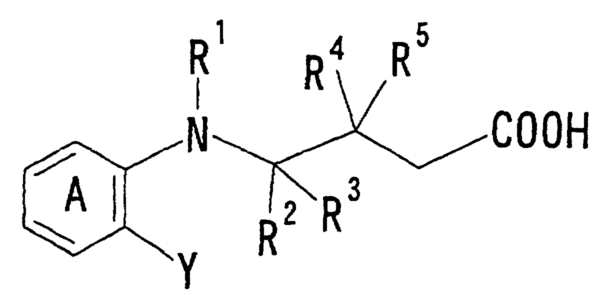 wherein Y is an electron-withdrawing group; ring A is an optionally substituted benzene ring; R¹ is an optionally substituted hydrocarbon group, an optionally substituted acyl group, or a substituted sulfonyl group; R², R³, R⁴, and R⁵ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R¹ and R², R¹ and R⁴, R² and R³, R⁴ and R⁵, or R² and R⁴ may form a ring, or a salt thereof, are novel compounds and useful as intermediates for synthesizing anilide derivatives described, for example, in WO99/32100, WO99/32468, and Japanese Patent Application No. 11-170345, by their being subjected to esterification and ring-closing reaction according to the reactions described above in (16) and (21), respectively, followed by the optional hydrolysis of the resulting esters, and then condensation with aniline derivatives according to the method known per se.

In addition, the compound of the formula: 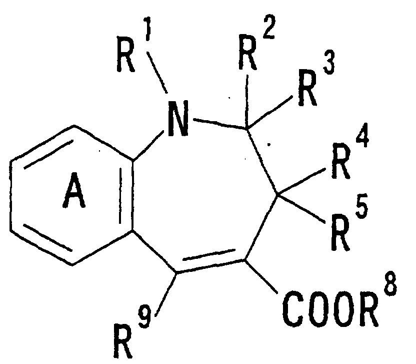 wherein each variable is as defined above, or a salt thereof, which is obtained by the reaction described above in (26), can be converted into a free acid, a salt thereof, or a reactive derivative thereof, and then subjected to condensation with the compound of the formula: 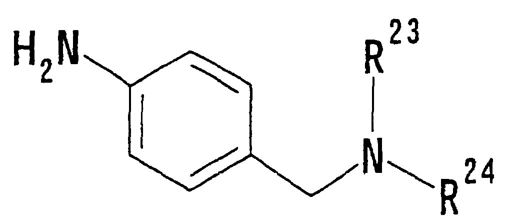 wherein R²³ and R²⁴ are independently an optionally substituted hydrocarbon group or an optionally substituted heterocyclic group, or a salt thereof (well-known condensation described, for example, in WO99/32100 or WO99/32468) to give a compound of the formula: 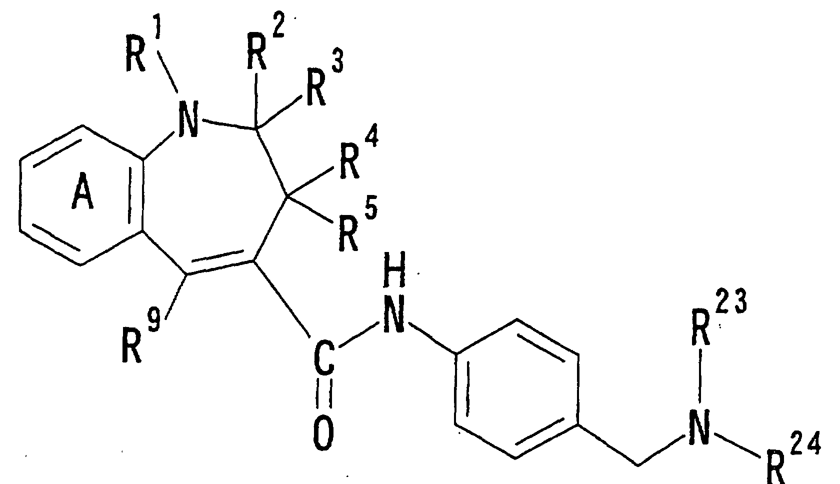 wherein each variable is as defined above, or a salt thereof.

In the above formulas, the "optionally substituted heterocyclic group" represented by R²³ or R²⁴ may include the same groups as the "optionally substituted heterocyclic group" represented by R², R³, R⁴, R⁵, R⁶, or R⁷.

In the above formulas, the R²³ may preferably be an optionally substituted straight chain hydrocarbon group (*e.g.,* optionally substituted alkyl, optionally substituted alkenyl), more preferably an optionally substituted lower C₁₋₆ alkyl group, and particularly preferred is a methyl group.

The R²⁴ may preferably be an optionally substituted alicyclic hydrocarbon group (*e.g.*, optionally substituted cycloalkyl or cycloalkenyl; preferably, an optionally substituted lower C₃₋₈ cycloalkyl group; more preferably cyclohexyl) or an optionally substituted alicyclic heterocyclic group (preferably, an optionally substituted saturated alicyclic heterocyclic group (preferably, 6-membered cyclic group); more preferably, optionally substituted tetrahydropyranyl, optionally substituted tetrahydrothiopyranyl, or optionally substituted piperidyl; particularly preferably, tetrahydropyranyl).

### The Best Mode for Carrying Out the Invention

The present invention will be further illustrated by reference to the following Reference Examples and Examples; however, these are only illustrative and do not limit the present invention in any way.

### Examples

### Example 1

### Synthesis of 4-(4-bromo-2-formyl-N-methylanilino)-butyric acid

To a suspension of 5-bromo-2-fluorobenzaldehyde (20.3 g) and 4-methylaminobutyric acid hydrochloride (18.4 g) in DMSO/water(100 ml/100 ml) was slowly add sodium carbonate (25.4 g), followed by stirring at 105°C to 110°C for 3.5 hours. The mixture was cooled to 50°C to 60°C, at which temperature 6N hydrochloric acid was added dropwise to adjust the pH to 3. The mixture was extracted with ethyl acetate (200 ml + 100 ml), and the organic layer was washed with a saturated aqueous sodium chloride solution (40 ml × 2) and water (40ml). The organic layer was concentrated, and the concentrate was then dissolved in IPE (50 ml), to which n-hexane (50 ml) was added dropwise. The precipitates were filtered out and washed with IPE/n-hexane (16 ml/4 ml). The filter cake was dried under reduced pressure at room temperature for 4 hours to give 4-(4-bromo-2-formyl-N-methylanilino)butyric acid (25.9 g, 86% yield) as yellow crystals.

¹H-NMR (CDCl₃, δ, 300 MHz): 1.95 (2H, tt, J = 6.5, 7.4 Hz), 2.38 (2H, t, J = 6.5 Hz), 2.88 (3H, s), 3.17 (2H, t, J = 7.4 Hz), 7.01 (1H, d, J = 8.7 Hz), 7.55 (1H, dd, J = 8.7, 2.5 Hz), 7.87 (1H, d, J = 2.5 Hz), 10.16 (1H, s).

### Example 2

### Synthesis of 4-(4-bromo-2-folmyl-N-methylanilino)-butyric acid ethyl ester

To a suspension of 4-(4-bromo-2-folmyl-N-methylanilino)butyric acid (1.00 g) and potassium carbonate (0.51 g) in DMF (5ml) was added ethyl iodide (0.26 ml), followed by stirring at room temperature for 24 hours. To the reaction mixture was added water, followed by extraction with ethyl acetate. The organic layer was washed with a saturated aqueous sodium chloride solution, dried over anhydrous sodium sulfate, and then concentrated. The concentrate was purified on a silica gel column (n-hexane/ethyl acetate (4/1)) to give 4-(4-bromo-2-formyl-N-methylanilino)butyric acid ethyl ester (0.84g, 77% yield) as an oil.

¹H-NMR (CDCl₃, δ, 300 MHz): 1.24 (3H, t, J = 7.1 Hz), 1.93 (2H, tt, J = 7.1, 7.7 Hz), 2.30 (2H, t, J = 7.1 Hz), 2.88 (3H, s), 3.15 (2H, t, J = 7.7 Hz), 4.12 (2H, q, J = 7.1 Hz), 7.01 (1H, d, J = 8.8 Hz), 7.55 (1H, dd, J = 8.8, 2.5 Hz), 7.87 (1H, d, J = 2.5 Hz), 10.17 (1H, s).

### Example 3

### Synthesis of 4-(4-bromo-2-formyl-N-methylanilino)-butyric acid ethyl ester

To a suspension of 4-(4-bromo-2-formyl-N-methylanilino)butyric acid (1.00 g) and potassium carbonate (0.55 g) in DMF (5 ml) was added ethyl bromide (0.30 ml), followed by stirring at room temperature for 10 hours. To the reaction mixture was added water, followed by extraction with ethyl acetate. The organic layer was washed with a saturated aqueous sodium chloride solution, dried over anhydrous sodium sulfate, and then concentrated. The concentrate was purified on a silica gel column (n-hexane/ethyl acetate (4/1)) to give 4-(4-bromo-2-formyl-N-methylanilino)butyric acid ethyl ester (0.98 g, 90% yield) as an oil.

### Example 4

### Synthesis of 4-(4-bromo-2-formyl-N-methylanilino)-butyric acid methyl ester

To a suspension of 4-(4-bromo-2-formyl-N-methylanilino)butyric acid (2.00 g) and potassium carbonate (1.01 g) in DMF (10 ml) was added ethyl iodide (0.46 ml), followed by stirring at room temperature for 2 hours. To the reaction mixture was added water, followed by extraction with ethyl acetate. The organic layer was washed with a saturated aqueous sodium chloride solution, dried over anhydrous sodium sulfate, and then concentrated. The concentrate was purified on a silica gel column (n-hexane/ethyl acetate (4/1)) to give 4-(4-bromo-2-formyl-N-methylanilino)butyric acid methyl ester (1.76 g, 84% yield) as an oil.

¹H-NMR (CDCl₃, δ, 300 MHz): 1.94 (2H, tt, J = 7.2, 7.5 Hz), 2.32 (2H, t, J = 7.2 Hz), 2.87 (3H, s), 3.15 (2H, t, J = 7.5 Hz), 3.66 (3H, s), 7.01 (1H, d, J = 8.8 Hz), 7.55 (1H, dd, J = 8.8, 2.5 Hz), 7.87 (1H, d, J = 2.5 Hz), 10.17 (1H, s) .

### Example 5

### Synthesis of 7-bromo-1-methyl-2,3-dihydro-1-benzaze-pine-4-carboxylic acid ethyl ester

To a solution of 4-(4-bromo-2-formyl-N-methylanilino)-butyric acid ethyl ester (0.97 g) in diethyl carbonate (20 ml) was added a 20% ethanolic sodium ethylate solution (1.21 g), followed by stirring at room temperature for 4 hours. The mixture was neutralized with 1N hydrochloric acid, followed by extraction with ethyl acetate. The organic layer was washed with water, dried over anhydrous sodium sulfate, and then concentrated to give 7-bromo-1-methyl-2,3-dihydro-1-benzazepine-4-carboxylic acid ethyl ester (0.83 g, 90% yield) as yellow crystals.

¹H-NMR (CDCl₃, δ, 300 MHz): 1.34 (3H, t, J = 7.0 Hz), 2.84 (2H, t, J = 4.5 Hz), 3.01 (3H, s), 3.22 (2H, t, J = 4.5 Hz), 4.25 (2H, q, J = 7.0 Hz), 6.65 (1H, d, J = 8.9 Hz), 7.26 (1H, dd, J = 8.9, 2.4 Hz), 7.42 (1H, d, J = 2.4 Hz), 7.56 (1H, s).

### Example 6

### Synthesis of 7-bromo-1-methyl-2,3-dihydro-1-benzaze-pine-4-carboxylic acid ethyl ester

To a suspension of 4-(4-bromo-2-formyl-N-methylanilino)butyric acid (1.00 g) and potassium carbonate (0.55 g) in DMF(3 ml) was added ethyl bromide (0.30 ml), followed by stirring at room temperature overnight. Then added were diethyl carbonate (6 ml) and a 20% ethanolic sodium ethylate solution (2.72 g), followed by stirring at 50°C for 1 hour. The mixture was neutralized with 2N hydrochloric acid, followed by extraction with ethyl acetate. The organic layer was washed with water, dried over anhydrous sodium sulfate, and then concentrated. The concentrate was purified on a silica gel column (n-hexane/ethyl acetate (4/1)) to give 7-bromo-1-methyl-2,3-dihydro-1-benzazepine-4-carboxylic acid ethyl ester (0.86 g, 83% yield) as yellow crystals.

### Example 7

### Synthesis of 7-bromo-1-methyl-2,3-dihydro-1-benzazepine-4-carboxylic acid methyl ester

To a solution of 4-(4-bromo-2-formyl-N-methylanilino)-butyric acid methyl ester (0.50 g) in dimethyl carbonate (10 ml) was added a 28% methanolic sodium methylate solution (0.37 g), followed by stirring at room temperature for 5.5 hours. The mixture was neutralized with 1N hydrochloric acid, followed by extraction with ethyl acetate. The organic layer was washed with water, dried over anhydrous sodium sulfate, and then concentrated. To the concentrate was added IPE (5ml), followed by stirring at room temperature for 0.5 hour. The crystals were filtered out, washed with IPE (5mL), and dried under reduced pressure at room temperature for 4 hours to give 7-bromo-1-methyl-2,3-dihydro-1-benzazepeine-4-carboxylic acid methyl ester (0.39 g, 83% yield) as yellow crystals.

¹H-NMR (CDCl₃, δ, 300 MHz): 2.85 (2H, t, J = 5.1 Hz), 3.00 (3H, s), 3.23 (2H, t, J = 5.1 Hz), 3.80 (3H, s) , 6.67 (1H, d, J = 8.8 Hz), 7.26 (1H, dd, J = 8.8, 2.4 Hz), 7.42 (1H, d, J = 2.4 Hz), 7.56 (1H, s).

### Example 8

### Synthesis of 4-(4-bromo-2-formyl-N-butylanilino)-butyric acid

A solution of 1-butyl-2-pyrrolidone (3.75 ml) in concentrated hydrochloric acid (7 ml) was refluxed for 24 hours. The pH was adjusted to 6 with 6N aqueous sodium hydroxide. Further added were water (8 ml), sodium carbonate (10.60 g), and a solution of 5-bromo-2-fluorobenzaldehyde (5.10 g) in DMSO (25 ml), followed by stirring at 105°C to 110°C for 5.5 hours. The mixture was cooled to 50°C to 60°C, at which temperature 6N hydrochloric acid was added dropwise to adjust the pH to 6. The aqueous layer was washed with IPE, and the organic layer was extracted with 2N aqueous sodium hydroxide (25 ml). The aqueous layer was adjusted to pH = 1 and extracted with ethyl acetate/THF (4/1, 25 ml × 3), after which the organic layer was washed with a saturated aqueous sodium chloride solution (25 ml × 2). The organic layer was concentrated, and the concentrate was then purified on a silica gel column (n-hexane/ethyl acetate (2/1, then 1/1)) to give 4-(4-bromo-2-formyl-N-butylanilino)butyric acid (7.20 g, 84% yield) as an oil.

¹H-NMR (CDCl₃, δ, 300 MHz): 0.85 (3H, t, J = 7.3 Hz), 1.23-1.28 (2H, m), 1.43-1.48 (2H, m), 1.86 (2H, tt, J = 7.1, 7.3 Hz), 2.34 (2H, t, J = 7.1 Hz), 3.11 (2H, t, J = 7.6 Hz), 3.19 (2H, t, J = 7.3 Hz), 7.06 (1H, d, J = 8.7 Hz), 7.57 (1H, dd, J = 8.7, 2.5 Hz), 7.90 (1H, d, J = 2.5 Hz), 10.22 (1H, s).

### Example 9

### Synthesis of 7-bromo-1-butyl-2,3-dihydro-1-benzazepine-4-carboxylic acid ethyl ester

To a suspension of 4-(4-bromo-2-formyl-N-butylanilino)butyric acid (1.00 g) and potassium carbonate (0.49 g) in DMF(3 ml) was added ethyl bromide (0.26 ml), followed by stirring at room temperature for 24 hours. Then added were diethyl carbonate (6 ml) and a 20% ethanolic sodium ethylate solution (2.39 g), followed by stirring at 50°C for 1.5 hours. The mixture was neutralized with 2N hydrochloric acid, followed by extraction with ethyl acetate. The organic layer was washed with water and concentrated. The concentrate was then purified on a silica gel column (n-hexane/ethyl acetate (10/1)) to give 7-bromo-1-butyl-2,3-dihydro-1-benzazepine-4-carboxylic acid ethyl ester (0.83 g, 81% yield) as an oil.

¹H-NMR (CDCl₃, δ, 300 MHz): 0.96 (3H, t, J = 7.3 Hz), 1.28-1.37 (5H, m), 1.60-1.66 (2H, m), 2.79 (2H, t, J = 4.4 Hz), 3.19-3.29 (4H, m), 4.25 (2H, q, J = 7.2 Hz), 6.68 (1H, d, J = 8.9 Hz), 7.23 (1H, dd, J = 8.9, 2.4 Hz), 7.41 (1H, d, J = 2.4 Hz), 7.55 (1H, s).

### Reference Example 1

### Synthesis of 1-propyl-2-pyrrolidone

A suspension of 2-pyrrolidone (40.0 g), propionaldehyde (109.0 g), anhydrous sodium sulfate (106.0 g), and 20% Pd-C (wet, 10.0 g) in ethyl acetate (470 ml) was stirred under an atmosphere of 4 MPa hydrogen at 100°C for 4 hours. After cooling to room temperature, insolubles were filtered and washed with ethyl acetate. The mother liquid was concentrated, and the concentrate was then distilled under reduced pressure (72°C to 77°C/5 mmHg) to give 1-propyl-2-pyrrolidone (56.2 g, 94% yield) as a colorless oil.

¹H-NMR (CDCl₃, δ, 300 MHz): 0.90 (3H, t, 7.4 Hz), 1.49-1.61 (2H, m), 1.97-2.05 (2H, m), 2.40 (2H, t, J = 8.3 Hz), 3.25 (2H, t, J = 7.6 Hz), 3.39 (2H, t, J = 7.1 Hz).

### Reference Example 2

### Synthesis of 1-propyl-2-pyrrolidone

A suspension of potassium hydroxide (36.3g) and tetrabutylammonium bromide (9.5 g) in toluene (250 ml) was refluxed using Dean Stark apparatus. Under the same conditions, a solution of 2-pyrrolidone (50.0 g) and propane bromide (94.0 g) in toluene (50 ml) was added dropwise over 2 hours, followed by stirring for 1.5 hours. After cooling to room temperature, insolubles were filtered. The filtrate was washed with a saturated aqueous sodium chloride solution/water (100 ml/100 ml), dried over anhydrous sodium sulfate, and then concentrated. The concentrate was then distilled under reduced pressure to give 1-propyl-2-pyrrolidone (41.5 g, 56% yield) as a colorless oil.

### Reference Example 3

### Synthesis of 4-propylaminobutyric acid

To a solution of 1-propyl-2-pyrrolidone (11.9 g) in water (150 ml) was added barium hydroxide octahydrate (53.25 g), followed by heating under reflux for 24 hours. After cooling, dry ice was added to precipitate insolubles. After filtration, insolubles were precipitated by the addition of sulfuric acid to the mother liquid. The insolubles were filtered out, and the solvent was then distilled out to give 4-propylaminobutyric acid (9.8 g, 72% yield) as a white solid.

¹H-NMR (D₂O, δ, 300 MHz): 0.69 (3H, t, J = 7.4 Hz), 1.47-1.40 (2H, m), 1.67-1.60 (2H, m), 2.01 (2H, t, J = 7.2 Hz), 2.82-2.69 (4H, m).

### Example 10

### Synthesis of 4-(4-bromo-2-formyl-N-propylanilino)-butyric acid

To a solution of 4-propylaminobutyric acid (5.8 g) in DMSO/water (40 ml/40 ml) were added 5-bromo-2-fluorobenzaldhyde (4.06 g) and sodium carbonate (6.36 g), followed by heating to reflux for 6 hours. After cooling, water (40ml) was added, and the mixture was washed with 50ml of IPE. The organic layer was extracted with 2N aqueous sodium hydroxide (30 ml). The combined aqueous layer was adjusted to around pH = 3, and extracted with ethyl acetate/THF (50 ml/50 ml). The aqueous layer was extracted with ethyl acetate (50 ml × 2). The combined organic layer was washed with a saturated aqueous sodium chloride solution, and the solvent was then distilled out to give 4-(4-bromo-2-formyl-N-propylanilino)butyric acid (5.4 g, 82% yield) as an oil.

¹H-NMR (CDCl₃, δ, 300 MHz): 0.83 (3H, t, J = 7.4 Hz), 1,54-1.47 (2H, m), 1.88-1.81 (2H, m), 2.34 (2H, t, J = 7.2 Hz), 3.11-3.06 (2H, m), 3.22-3.18 (2H, m), 7.07 (1H, d, J = 8.7 Hz), 7.57 (1H, dd, J = 8.7, 2.5 Hz), 7.90 (1H, d, J = 2.5 Hz), 10.22 (1H, s).

### Example 11

### Synthesis of 4-(4-bromo-2-formyl-N-propylanilino)-butyric acid

A solution of 1-propyl-2-pyrrolidone (2.54 g) in a 4N aqueous sodium hydroxide solution (10ml) was stirred for 8 hours, while heating under reflux. After cooling, concentrated hydrochloric acid (3.3 ml) was added. Then added were a solution of 5-bromo-2-fluorobenzaldehyde (2.03 g) in DMSO (20 ml) and sodium carbonate (4.24 g), followed by heating under reflux for 7 hours. After cooling, water (10 ml) and THF (10 ml) were added, and the mixture was adjusted to pH = 3.5 with 6N hydrochloric acid. The mixture was extracted with ethyl acetate (20 ml) and then further extracted with ethyl acetate/THF (20 ml/10 ml). The combined organic layer was washed with a saturated aqueous sodium chloride solution/water (10 ml/10 ml × 2) and then concentrated. The concentrate was purified on a silica gel column (n-hexane/ethyl acetate (4/1, then 2/1)) to give 4-(4-bromo-2-formyl-N-propylanilino)butyric acid (2.90 g, 88% yield) as an oil.

### Example 12

### Synthesis of 4-(4-bromo-2-formyl-N-propylanilino)-butyric acid ethyl ester

To a suspension of 4-(4-bromo-2-formyl-N-propylanilino)butyric acid (5.4 g) and potassium carbonate (2.74 g) in DMF (25 ml) was added ethyl bromide (1.5 ml), followed by stirring at room temperature for 24 hours. Water (30 ml) was added, followed by extraction with ethyl acetate (50 ml). Further, the aqueous layer was extracted with ethyl acetate (25 ml × 2). The combined organic layer was washed with a saturated aqueous sodium chloride solution and then concentrated. The concentrate was purified on a silica gel column (n-hexane, then n-hexane/ethyl acetate (20/1, then 15/1, then 10/1)) to give 4-(4-bromo-2-formyl-N-propylanilino)butyric acid ethyl ester (3.74 g, 64% yield) as an oil.

¹H-NMR (CDCl₃, δ, 300 MHz): 0.83 (3H, t, J = 7.4 Hz), 1.23 (3H, t, J = 7.1 Hz), 1.52-1.49 (2H, m), 1.84-1.79 (2H, m), 2,27 (2H, t, J = 7.2 Hz), 3.11-3.06 (2H, m), 3.20-3.16 (2H, m), 4.10 (2H, q, J = 7.1 Hz), 7.07 (1H, d, J = 8.7 Hz), 7.56 (1H, dd, J = 8.7, 2.6 Hz), 7.90 (1H, d, J = 2.6 Hz), 10.22 (1H, s).

### Example 13

### Synthesis of 7-bromo-1-propyl-2,3-dihydro-1-benzazepine-4-carboxylic acid ethyl ester

To a solution of 4-(4-bromo-2-formyl-N-propylanilino)-butyric acid ethyl ester (3.00 g) in diethyl carbonate (60 ml) was added dropwise a 20% ethanolic sodium ethoxide solution (3.44 g), followed by stirring at room temperature for 4 hours. Under ice-cooling, the mixture was adjusted to pH = 2 with 1N hydrochloric acid (10 ml), and the layers were separated. Further, the aqueous layer was extracted with ethyl acetate (30 ml). The combined organic layer was washed with a saturated aqueous sodium chloride solution and then concentrated. The concentrate was purified on a silica gel column (n-hexane, then n-hexane/ethyl acetate (10/1, then 7/1)) to give 7-bromo-1-propyl-2,3-dihydro-1-benzazepine-4-carboxylic acid methyl ester (2.34 g, 82% yield) as an oil.

¹H-NMR (CDCl₃, δ, 300 MHz): 0.94 (3H, t, J = 7.4 Hz), 1.34 (3H, t, J = 7.1 Hz), 1.72-1.64 (2H, m), 2.81-2.78 (2H, m), 3.25-3.19 (4H, m), 4.25 (2H, q, J = 7.1 Hz), 6.68 (1H, d, J = 8.9 Hz), 7.22 (1H, dd, J = 8.9, 2.4 Hz), 7.41 (1H, d, J = 2.4 Hz), 7.55 (1H, s).

### Example 14

### Synthesis of 7-bromo-1-propyl-2,3-dihydro-1-benzazepine-4-carboxylic acid ethyl ester

To a suspension of 4-(4-bromo-2-formyl-N-propylanilino)butyric acid (4.00 g) and potassium carbonate (2.02 g) in DMF (12 ml) was added ethyl bromide (1.1 ml), followed by stirring at room temperature for 6 hours. Then added was diethyl carbonate (24 ml), to which a 20% ethanolic sodium ethoxide solution (10.00 g) was added dropwise, followed by stirring at 60°C for 1 hour. Under ice-cooling, the mixture was adjusted to pH = 2 with 1N hydrochloric acid (50 ml), and the layers were separated. Further, the aqueous layer was extracted with ethyl acetate (50 ml). The combined organic layer was washed with a saturated aqueous sodium chloride solution and then concentrated. The concentrate was purified on a silica gel column (n-hexane, then n-hexane/ethyl acetate (10/1, then 7/1)) to give 7-bromo-1-propyl-2,3-dihydro-1-benzazepiene-4-caboxylic acid ethyl ester (3.41 g, 83% yield) as an oil.

### Example 15

### Synthesis of 4-[4-(4-butoxyethoxyphenyl)-2-formyl-N-propylanilino]butyric acid

Under an argon atmosphere, magnesium (0.076 g) was suspended in THF (6.75 ml), to which a solution of 4-butoxyethoxybromobenzene (0.83 g) in THF (2.25 ml) was added dropwise, while heating under reflux, followed by stirring at the same temperature for 2 hours. After cooling, a solution of trimethoxyborane (0.32 g) in THF (2.25 ml) was added dropwise at -10°C or below. After stirring at the same temperature for 0.5 hour, the mixture was stirred at room temperature for 1 hour. The mixture was concentrated under reduced pressure, and to the resulting residue were added toluene (6 ml), ethanol (2 ml), palladium acetate (3.4 mg), and triphenylphosphine (16 mg), followed by stirring at room temperature for 0.5 hour. 4-(4-Bromo-2-formyl-N-propylanilino)butyric acid (0.5 g) and an aqueous solution of potassium carbonate (1.1 g) in water (2 ml) were added. While heating under reflux, the mixture was stirred for 3.5 hours. After cooling, 2N hydrochloric acid (11 ml) was added dropwise, and the pH was adjusted to 3 using a 2N aqueous sodium hydroxide solution. After the layers were separated, toluene (10 ml) was added to the aqueous layer, followed by extraction. The organic layers were combined, washed with a 20% aqueous sodium chloride solution (7.5 ml × 2), and concentrated under reduced pressure. The resulting residue was purified on a silica gel column (n-hexane/ethyl acetate = 2 : 1) to give 4-(4-butoxyethoxyphenyl-2-formyl-N-propylanilino)butyric acid (0.5 g, 75.5% yield).

¹H-NMR (CDCl₃, δ, 300 MHz): 0.84 (3H, t, J = 7.4 Hz), 0.93 (3H, t, J = 7.3 Hz), 1.3-1.45 (2H, m), 1.50-1.68 (4H, m), 1,80-1.95 (2H, m), 2.30-2.38 (2H, m), 3.09-3.14 (2H, m), 3.53-3.58 (2H, m), 3.55 (2H, t, J = 6.7 Hz), 3.78-3.83 (2H, m), 4.13-4.18 (2H, m), 6.99 (2H, d, J = 8.7 Hz), 7.22 (1H, d, J = 8.5 Hz), 7.51 (2H, d, J = 8.7 Hz), 7.69 (1H, dd, J = 8.5, 2.4 Hz), 7.99 (1H, d, J = 2.4 Hz), 10.37(1H, s).

### Reference Example 4

### Synthesis of 5-(4-butoxyethoxyphenyl)-2-fluorobenzaldehyde

Under an argon atmosphere, magnesium (4.9 g) was suspended in THF (270 ml), to which a solution of 4-butoxyethoxybromobenzene (53.8 g) in THF (90 ml) was added dropwise, while heating under reflux, followed by stirring at the same temperature for 2 hours. After cooling, a solution of trimethoxyborane (20.5 g) in THF (90 ml) was added dropwise at -10°C. After stirring at the same temperature for 0.5 hour, the mixture was stirred at room temperature for 1 hour. Palladium acetate (221 mg) and triphenylphosphine (1.03 g) were added, and the mixture was stirred at room temperature for 0.5 hour. 5-Bromo-2-fluorobenzaldehyde (20 g) and an aqueous solution of potassium carbonate (71.5 g) in water (85 ml) were added. The mixture was stirred for 2.5 hours, while heating under reflux. After cooling, 2N hydrochloric acid (450 ml) was added dropwise, and the layers were separated. To the aqueous layer was added toluene (450ml), followed by extraction. The organic layers were combined, and washed with 2N hydrochloric acid (300 ml), a 2N aqueous sodium hydroxide solution (300 ml × 2), and a 20% aqueous sodium chloride solution (300 ml x 3). To the organic layer was added active carbon (1 g), followed by stirring at room temperature for 20 hours. The active carbon was filtered out, followed by concentration under reduced pressure. The resulting residue was purified on a silica gel column (n-hexane/ethyl acetate = 5 : 1) to give 5-(4-butoxyethoxyphenyl)-2-fluorobenzaldehyde (28.2 g, 90.5% yield).

¹H-NMR (CDCl₃, δ, 300 MHz): 0.93 (3H, t, J = 7.3 Hz), 1.26-1.46 (2H, m), 1.56-1.66 (2H, m), 3.55 (2H, t, J = 6.6 Hz), 3.78-3.81 (2H, m), 4.12-4.18 (2H, m), 7.00 (2H, dd, J = 6.6, 2.1 Hz), 7.21 (1H, dd, J = 9.9, 8.7 Hz), 7.49 (2H, dd, J = 6.6, 2.1 Hz), 7.47-7.79 (1H, m), 8.02 (1H, dd, J = 6.5, 2.5 Hz), 10.40 (1H, s).

### Example 16

### Synthesis of 4-[4-(4-butoxyethoxyphenyl)-2-formyl-N-propylanilino]butyric acid

1-Propyl-2-pyrrolidone (0.8 g) was dissolved in a 2N aqueous sodium hydroxide solution (6.3 ml), followed by stirring for 8 hours, while heating under reflux. After cooling, 12N hydrochloric acid (1 ml) was added for neutralization, and a solution of sodium carbonate (1.34 g) and 5-(4-butoxyethoxyphenyl)-2-fluorobenzaldehyde (1 g) in dimethyl sulfoxide (7.3 ml) was added. The mixture was stirred for 49 hours, while heating under reflux. After cooling, 2N hydrochloric acid (12 ml) was added, and the pH was adjusted to 3 using a 2N aqueous sodium hydroxide solution. The mixture was extracted with toluene (10 ml × 2). The organic layer was washed with a 20% aqueous sodium chloride solution (10 ml × 2), dried over anhydrous magnesium sulfate, and then concentrated under reduced pressure. The resulting residue was purified on a silica gel column (n-hexane/ethyl acetate = 2 : 1) to give 4-(4-butoxyethoxyphenyl-2-formyl-N-propylanilino)butyric acid (0.2 g, 15% yield).

### Example 17

### 4-(2-Formyl-N-methylanilino)butyric acid

Using 2-fluorobenzaldehyde, the reaction was carried out in the same manner as described in Example 1 to give 4-(2-formyl-N-methylanilino)butyric acid in 78% yield as a brown oil.
EI-MS m/z: 221 (M⁺)
¹H-NMR (CDCl₃, δ, 300 MHz): 1.92 (2H, tt, J = 7.2, 7.3 Hz), 2.36 (2H, t, J = 7.2 Hz), 2.85 (3H, s), 3.15 (2H, t, J = 7.3 Hz), 7.06-7.10 (2H, m), 7.43-7.49 (1H, m), 7.75 (1H, dd, J = 7.7, 1.7 Hz), 10.23 (1H, s).
IR (neat, cm⁻¹): 2960, 1708, 1683, 1596.

### Example 18

### 4-(2-Formyl-4-nitro-N-methylanilino)butyric acid

Using 2-chloro-5-nitrobenzaldehyde, the reaction was carried out in the same manner as described in Example 1 to give 4-(2-formyl-4-nitro-N-methylanilino)butyric acid in 92% yield as yellow crystals.
m.p.: 100°C to 101°C.
Anal. for C₁₂H₁₄N₂O₅:
Calcd.: C, 54.13; H, 5.30; N, 10.52;
Found.: C, 54.06; H, 5.31; N, 10.43.
¹H-NMR (CDCl₃, δ, 300 MHz): 2.01 (2H, tt, J = 6.9, 7.5 Hz), 2.39 (2H, t, J = 6.9 Hz), 3.04 (3H, s), 3.46 (2H, t, J = 7.5 Hz), 7.2 (1H, d, J = 9.3 Hz), 8.19 (1H, dd, J = 9.3, 2.3 Hz), 8.57 (1H, d, J = 2.3 Hz), 9.95 (1H, s).
IR (KBr, cm⁻¹): 1693, 1598, 1315, 1257, 1228.

### Example 19

### 1-Methyl-2,3-dihydro-1-benzaldehyde-4-carboxylic acid ethyl ester

Using 4-(2-formyl-N-methylanilino)butyric acid, the reaction was carried out in the same manner as described in Example 6 to give 1-methyl-2,3-dihydro-1-benzaldehyde-4-carboxylic acid ethyl ester in 74% yield as a yellow oil.
EI-MS m/z: 231 (M⁺).
¹H-NMR (CDCl₃, δ, 300 MHz): 1.31 (3H, t, J = 7.1 Hz), 2.81 (2H, t, J = 4.3 Hz), 3.00 (3H, s) , 3.22 (2H, t, J = 4.9 Hz), 4.22 (2H, q, J = 7.1 Hz), 6.75-6.80 (2H, m), 7.16-7.30 (2H, m), 7.66 (1H, s).
IR (neat, cm⁻¹) : 1702, 1689, 1498, 1255, 1186.

### Example 20

### 1-Methyl-7-nitro-2,3-dihydro-1-benzazepine-4-carboxylic acid ethyl ester

Using 4-(2-formyl-4-nitro-N-methylanilino)butyric acid, the reaction was carried out in the same manner as described in Example 6 to give 1-methyl-7-nitro-2,3-dihydro-1-benzaldehyde-4-carboxylic acid ethyl ester in yield 72% as yellow crystals.
m.p.: 113°C to 114°C.
Anal. for C₁₄H₁₆N₂O₄:
Calcd.: C, 60.86; H, 5.84; N, 10.14;
Found.: C, 60.50; H, 5.84; N, 10.03.
¹H-NMR (CDCl₃, δ, 300 MHz): 1.32 (3H, t, J = 7.1 Hz), 2.86 (2H, t, J = 4.5 Hz), 3.13 (3H, s), 3.35 (2H, t, J = 4.9 Hz), 4.24 (2H, q, J = 7.1 Hz), 6.71 (1H, d, J = 9.3 Hz), 7.62 (1H, s), 8.01 (1H, dd, J = 9.3, 2.7 Hz), 8.22 (1H, d, J = 2.7 Hz).
IR (KBr, cm⁻¹): 1697, 1596, 1565, 1321, 1299, 1257, 1187.

### Example 21

### 7-[4-(Butoxyethoxyphenyl)]-1-propyl-2,3-dihydro-1-benzazepine-4-carboxylic acid ethyl ester

Using 4-[4-(butoxyethxyphenyl)-2-formyl-N-propylanilino]butyric acid, the reaction was carried out in the same manner as described in Example 14 to give 7-[4-(butoxyethoxyphenyl)]-1-propyl-2,3-dihydro-1-benzazepine-4-carboxylic acid ethyl ester in 76% yield as a yellow oil.

EI-MS m/z: 451 (M⁺).
¹H-NMR (CDCl₃, δ, 300 MHz): 0.93-1.00 (6H, m), 1.34-1.45 (5H, m), 1.57-1.65 (2H, m), 1.72-1.78 (2H, m), 2.82-2.86 (2H, m), 3.28-3.35 (4H, m), 3.57 (2H, t, J = 6.6 Hz), 3.82 (2H, t, J = 4.7 Hz), 4.18 (2H, t, J = 5.2 Hz), 4.29 (2H, q, J = 7.1 Hz), 6.88 (1H, d, J = 8.6 Hz), 7.00 (2H, d, J = 8.6 Hz), 7.41 (1H, dd, J = 8.6, 2.0 Hz), 7.51 (2H, d, J = 8.6 Hz), 7.53 (1H, d, J = 2.0 Hz), 7.77 (1H, s).
IR (neat, cm⁻¹): 1697, 1500, 1241, 1178.

### Example 22

### 4-(4-Bromo-2-formyl-N-isopropylanilino)butyric acid

Using N-isopropyl-2-pyrrolidone, the reaction was carried out in the same manner as described in Example 11 to give 4-(4-bromo-2-formyl-N-isopropylanilino)butyric acid in 22% yield as yellow crystals.
m.p.: 96°C to 98°C.
Anal. for C₁₄H₁₈NO₃Br:
Calcd.: C, 51.23; H, 5.53; N, 4.27; Br, 24.35;
Found.: C, 51.01; H, 5.56; N, 4.22; Br, 24.61.
¹H-NMR (CDCl₃, δ, 300 MHz): 1.11 (6H, d, J = 6.6 Hz), 1.71-1.77 (2H, m) , 2.35 (2H, t, J = 7.1 Hz), 3.15 (2H, t, J = 6.7 Hz), 3.27-3.33 (1H, m), 7.11 (1H, d, J = 8.6 Hz), 7.60 (1H, dd, J = 8.6, 2.6 Hz), 7.95 (1H, d, J = 2.6 Hz), 10.21 (1H, s).
IR (KBr, cm⁻¹): 2969, 1700, 1675, 1473, 1213, 1178.

### Example 23

### 4-(4-Bromo-2-formyl-N-cyclohexylanilino)butyric acid

Using N-cyclohexyl-2-pyrrolidone, the reaction was carried out in the same manner as described in Example 11 to give 4-(4-bromo-2-formyl-N-cyclohexylanilino)butyric acid in 3% yield as a brown oil.
EI-MS m/z: 367(M⁺).
¹H-NMR (CDCl₃, δ, 300 MHz): 1.08-1.38 (5H, m) , 1.52-1.85 (7H, m), 2.33 (2H, t, J = 7.0 Hz), 2.74-2.88 (1H, m), 3.20 (2H, t, J = 7.3 Hz), 7.12 (1H, d, J = 8.7 Hz), 7.57 (1H, dd, J = 8.7, 2.5 Hz), 7.93 (1H, d, J = 2.5 Hz), 10.21 (1H, s).
IR (neat, cm⁻¹) : 2931, 2856, 1706, 1681, 1471.

### Example 24

### 4-(4-Bromo-2-formyl-N-allylanilino)butyric acid

Using N-allyl-2-pyrrolidone, the reaction was carried out in the same manner as described in Example 11 to give 4-(4-bromo-2-formyl-N-allylanilino)butyric acid in 50% yield as a brown oil.
El-MS m/z: 325 (M⁺).
¹H-NMR (CDCl₃, δ, 300 MHz): 1.85 (2H, tt, J = 7.1, 7.4 Hz), 2.35 (2H, t, J = 7.1 Hz), 3.19 (2H, t, J = 7.4 Hz), 3.71 (2H, d, J = 6.3 Hz), 5.17-5.23 (2H, m), 5.73-5.84 (1H, m), 7.05 (1H, d, J = 8.7 Hz), 7.58 (1H, dd, J = 8.7, 2.5 Hz), 7.90 (1H, d, J = 2.5 Hz), 10.24 (1H, s).
IR (neat, cm⁻¹): 2962, 1708, 1681, 1261, 1178.

### Example 25

### 4-[4-Bromo-2-formyl-N-(2-methoxybenzyl)anilino]butyric acid

To a solution of 2-methoxybenzaldehyde (3.0 g) in methanol (42 ml) were added 4-aminobutyric acid (2.3 g) and 1N sodium hydroxide (22 ml). A Kolben was purged with argon, to which 20% Pd-C (wet, 0.3 g) was added. The Kolben was purged with hydrogen, followed by stirring at room temperature for 4 hours. The Pd-C was filtered out, and the mixture was then washed with methanol. To the filtrate was added 6N hydrochloric acid (3.7 ml), followed by concentration to dryness. Then added were DMSO/water (29 ml/15 ml), 5-bromo-4-fluorobenzaldehyde (2.2 g), and sodium carbonate (4.7 g), followed by stirring for 5 hours, while heating under reflux. After cooling to room temperature, the pH was adjusted to about 3.5 with 6N hydrochloric acid. After extraction with ethyl acetate, the organic layer was washed with a saturated aqueous sodium chloride solution, dried over anhydrous sodium sulfate, and then concentrated. The concentrate was purified on a silica gel column (n-hexane/ethyl acetate = 2/1), and the effective fractions were concentrated to give 4-[4-bromo-2-formyl-N-(2-methoxybenzyl)anilino]butyric acid (3.5 g, 90% content, 70% yield) as a brown oil.
FAB-MS m/z: 428 (M⁺ + Na).
¹H-NMR (CDCl₃, δ, 300 MHz): 1.95 (2H, tt, J = 7.2, 6.9 Hz), 2.35 (2H, t, J = 7.2 Hz), 3.16 (2H, t, J = 6.9 Hz), 3.66 (3H, s), 4.28 (2H, s), 6.79-6.86 (2H, m), 6.98-7.06 (2H, m), 7.25 (1H, d, J = 8.7 Hz), 7.53 (1H, dd, J = 8.7, 2.5 Hz), 7.88 (1H, d, J = 2.5 Hz), 10.23 (1H, s).
IR (neat, cm⁻¹) : 1706, 1671, 1475, 1245.

### Example 26

### 7-Bromo-1-isopropyl-2,3-dihydro-1-benzazepine-4-carboxylic acid ethyl ester

Using 4-(4-bromo-2-formyl-N-isopropylanilino)butyric acid, the reaction was carried out in the same manner as described in Example 14 to give 7-bromo-1-isopropyl-2,3-dihydro-1-benzazepine-4-carboxylic acid ethyl ester in 73% yield as a brown oil.
EI-MS m/z: 337 (M⁺).
¹H-NMR (CDCl₃, δ, 300 MHz): 1.23 (6H, d, J = 6.6 Hz), 1.34 (3H, t, J = 7.1 Hz), 2.75-2.78 (2H, m), 3.12-3.15 (2H, m), 3.94-4.02 (1H, m), 4.25 (2H, q, J = 7.1 Hz), 6.70 (1H, d, J = 8.9 Hz), 7.24 (1H, dd, J = 8.9, 2.4 Hz), 7.42 (1H, d, J = 2.4 Hz), 7.54 (1H, s).
IR (neat, cm⁻¹): 1698, 1492, 1251, 1213, 1184.

### Example 27

### 7-Bromo-1-cyclohexyl-2,3-dihydro-1-benzazepine-4-carboxylic acid ethyl ester

Using 4-(4-bromo-2-formyl-N-cyclohexylanilino)butyric acid, the reaction was carried out in the same manner as described in Example 14 to give 7-bromo-1-cyclohexyl-2,3-dihydro-1-benzazepine-4-carboxylic acid ethyl ester in 44% yield as yellow crystals.

¹H-NMR (CDCl₃, δ, 300 MHz): 1.33 (3H, t, J = 7.1 Hz), 1.14-2.04 (10H, m), 2.73 (2H, m), 3.19 (2H, m), 3.49 (1H, m), 4.24 (2H, q, J = 7.1 Hz), 6.67 (1H, d, J = 8.9 Hz), 7.21 (1H, dd, J = 8.9, 2.3 Hz), 7.41 (1H, d, J = 2.3 Hz), 7.53 (1H, s).
IR (KBr,cm⁻¹): 2929, 1695, 1488, 1253, 1240.

### Example 28

### 1-Allyl-7-bromo-2,3-dihydro-1-benzazepine-4-carboxylic acid methyl ester

Using 4-(4-bromo-2-formyl-N-allylanilino)butyric acid, the reaction was carried out in the same manner as described in Example 14 to give 1-allyl-7-bromo-2,3-dihydro-1-benzazepine-4-carboxylic acid methyl ester in 50% yield as yellow crystals.
m.p.: 51°C to 53°C.
Anal. for C₁₅H₁₆NO₂Br:
Calcd.: C, 55.92; H, 5.01; N, 4.35; Br, 24.80;
Found.: C, 55.84; H, 4.94; N, 4.19; Br, 24.80.
¹H-NMR (CDCl₃, δ, 300 MHz): 2.78 (2H, t, J = 5.0 Hz), 3.21 (2H, d, J = 5.0 Hz), 3.80 (3H, s), 3.90 (2H, m), 5.21 (1H, d, J = 15.3 Hz), 5.25 (1H, d, J = 10.2 Hz), 5.58 (1H, m), 6.68 (1H, d, J = 8.8 Hz), 7.22 (1H, dd, J = 8.8, 2.4 Hz), 7.43 (1H, d, J = 2.4 Hz), 7.58 (1H, s).
IR (KBr, cm⁻¹) ; 1698, 1496, 1265, 1238, 1176.

### Example 29

### 7-Bromo-1-(2-methoxybenzyl)-2,3-dihydro-1-benzazepine-4-carboxylic acid ethyl ester

Using 4-[4-bromo-2-formyl-N-(2-methoxybenzyl)anilino]-butyric acid, the reaction was carried out in the same manner as described in Example 14 to give 7-bromo-1-(2-methoxybenzyl)-2,3-dihydro-1-benzazepine-4-carboxylic acid ethyl ester in 90% yield as a yellow oil.
EI-MS m/z: 415 (M⁺).
¹H-NMR (CDCl₃, δ, 300 MHz): 1.30 (3H, t, J = 7.1 Hz), 2.77 (2H, m), 3.26 (2H, m), 3.84 (3H, s), 4.23 (2H, q, J = 7.1 Hz), 4.47 (2H, s), 6.57 (1H, d, J = 8.8 Hz), 6.86-6.91 (2H, m), 7.03 (1H, d, J = 6.2 Hz), 7.13 (1H, dd, J = 8.8, 2.4 Hz), 7.26 (1H, d, J = 6.2 Hz), 7.43 (1H, d, J = 2.4 Hz), 7.60 (1H, s).
IR (neat, cm⁻¹): 1697, 1488, 1240, 1176.

### Industrial Applicability

According to the present invention, 2,3-dihydroazepine derivatives can be prepared in a few steps with safety by a process suitable for large-scale synthesis.

## Claims

1. A process for the preparation of a compound of the formula: 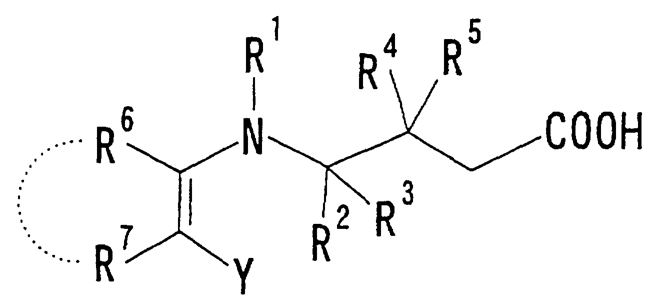 wherein each variable is as defined below, or a salt thereof, **characterized in that** a compound of the formula: 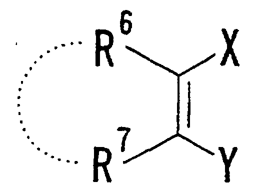 wherein X is a halogen atom; Y is an electron-withdrawing group; R⁶ and R⁷ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R⁶ and R⁷ may form a ring, or a salt thereof, is allowed to react with a compound of the formula: 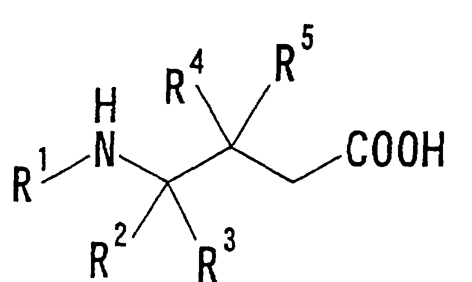 wherein R¹ is an optionally substituted hydrocarbon group, an optionally substituted acyl group, or an optionally substituted sulfonyl group; R², R³, R⁴, and R⁵ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R¹ and R², R¹ and R⁴, R² and R³, R⁴ and R⁵, or R² and R⁴ may form a ring, or a salt thereof.

2. The preparation process according to claim 1, wherein Y is an optionally substituted acyl group.

3. The preparation process according to claim 1, wherein R², R³, R⁴, and R⁵ are hydrogen atoms.

4. The preparation process according to claim 1, wherein R¹ is an optionally substituted hydrocarbon group.

5. A process for the preparation of a compound of the formula: 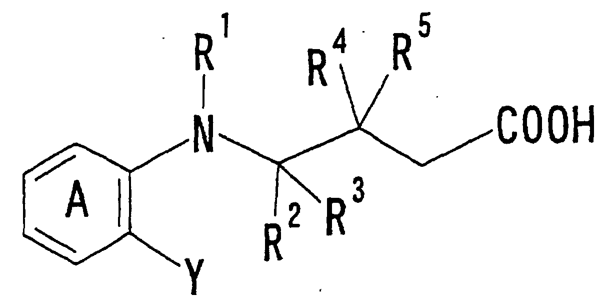 wherein each variable is as defined below, or a salt thereof, **characterized in that** a compound of the formula: 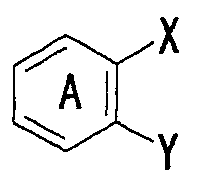 wherein X is a halogen atom; Y is an electron-withdrawing group; and ring A is an optionally substituted benzene ring, or a salt thereof, is allowed to react with a compound of the formula: 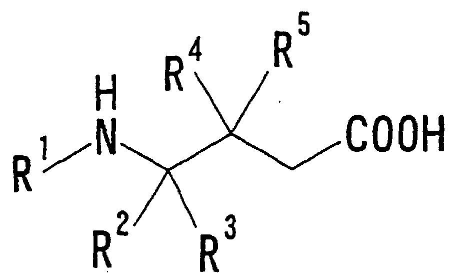 wherein R¹ is an optionally substituted hydrocarbon group, an optionally substituted acyl group, or an optionally substituted sulfonyl group; R², R³, R⁴, and R⁵ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R¹ and R², R¹ and R⁴, R² and R³, R⁴ and R⁵, or R² and R⁴ may form a ring, or a salt thereof.

6. The preparation process according to claim 1, **characterized in that** a compound of the formula: 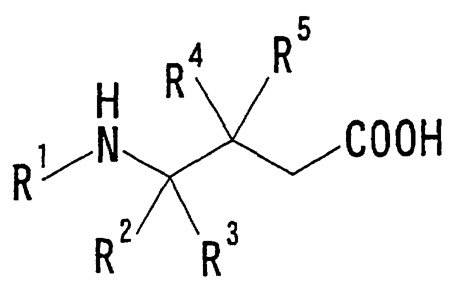 wherein each variable is as defined in claim 1, or a salt thereof, is used, which is obtained by hydrolyzing a compound of the formula: 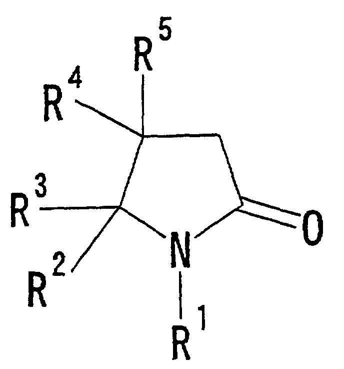 wherein each variable is as defined in claim 1, or a salt thereof.

7. The preparation process according to claim 6,
**characterized in that** the compound of the formula: 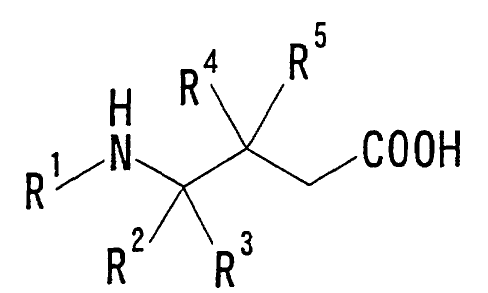 wherein each variable is as defined in claim 1, or a salt thereof, is subjected, without being isolated, to the reaction with the compound of the formula: 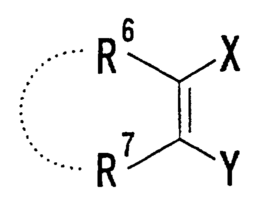 wherein each variable is as defined in claim 1, or a salt thereof.

8. A compound of the formula: 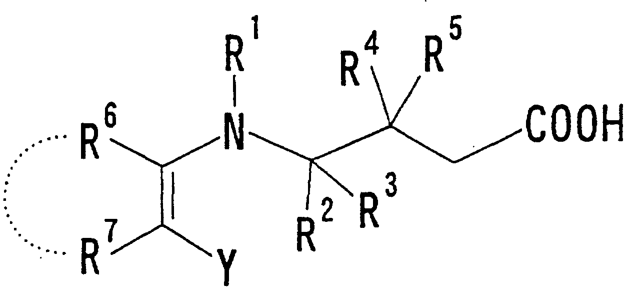 wherein Y is an electron-withdrawing group; R¹ is an optionally substituted hydrocarbon group, an optionally substituted acyl group, or an optionally substituted sulfonyl group; R², R³, R⁴, R⁵, R⁶, and R⁷ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R¹ and R², R¹ and R⁴, R² and R³, R⁴ and R⁵, R² and R⁴, or R⁶ and R⁷ may form a ring, or a salt thereof.

9. The compound according to claim 8, wherein Y is an optionally substituted acyl group.

10. The compound according to claim 8, wherein R², R³, R⁴, and R⁵ are hydrogen atoms.

11. The compound according to claim 8, wherein R¹ is an optionally substituted hydrocarbon group.

12. A compound of the formula: 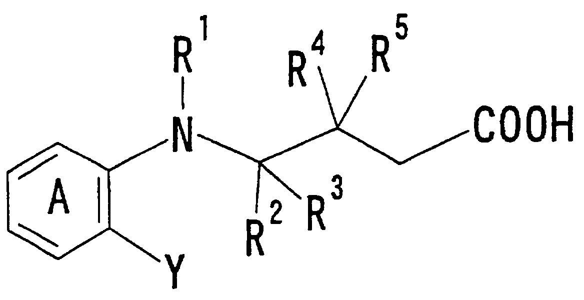 wherein Y is an electron-withdrawing group; ring A is an optionally substituted benzene ring; R¹ is an optionally substituted hydrocarbon group, an optionally substituted acyl group, or an optionally substituted sulfonyl group; R², R³, R⁴, and R⁵ are independently a hydrogen atom, a halogen atom, an optionally substituted amino group, an optionally substituted hydroxyl group, an optionally substituted thiol group, an optionally substituted hydrocarbon group, or an optionally substituted heterocyclic group; or R¹ and R², R¹ and R⁴, R² and R³, R⁴ and R⁵, or R² and R⁴ may form a ring, or a salt thereof.

13. The compound according to claim 12, wherein Y is an optionally substituted acyl group.

14. The compound according to claim 12, wherein R², R³, R⁴, and R⁵ are hydrogen atoms.

15. The compound according to claim 12, wherein R¹ is an optionally substituted hydrocarbon group.

16. A process for the preparation of a compound of the formula: 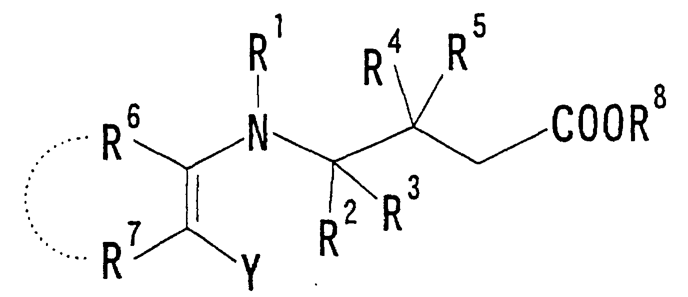 wherein R⁸ is an optionally substituted hydrocarbon group and the other variables are as defined below, or a salt thereof, **characterized in that** a compound of the formula: 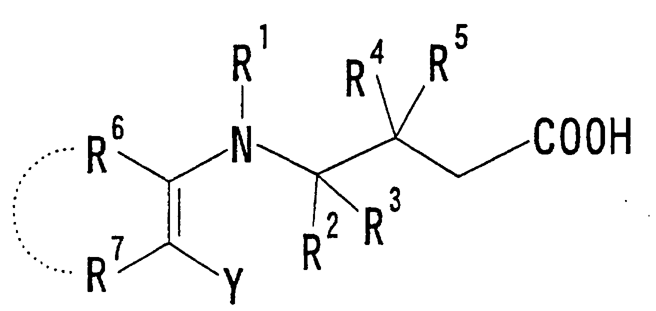 wherein each variable is as defined in claim 1, or a salt thereof, which is obtained by the preparation process according to claim 1, is subjected to esterification.

17. The preparation process according to claim 16, wherein Y is an optionally substituted acyl group.

18. The preparation process according to claim 16, wherein R², R³, R⁴, and R⁵ are hydrogen atoms.

19. The preparation process according to claim 16, wherein R¹ is an optionally substituted hydrocarbon group.

20. A process for the preparation of a compound of the formula: 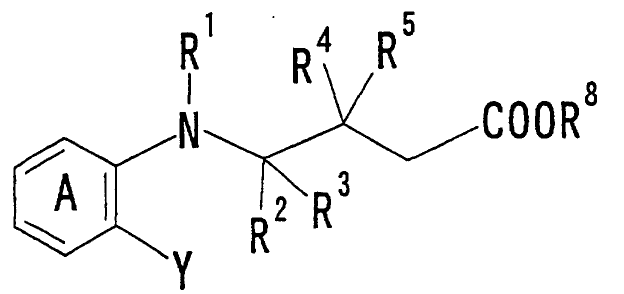 wherein R⁸ is an optionally substituted hydrocarbon group and the other variables are as defined below, or a salt thereof, **characterized in that** a compound of the formula: 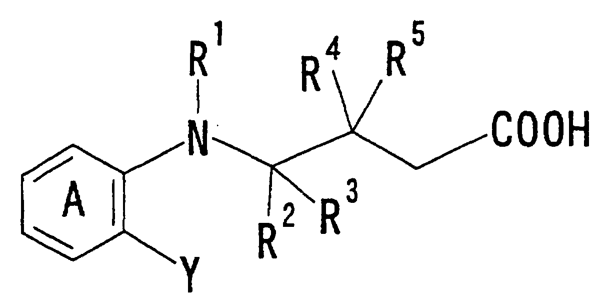 wherein each variable is as defined in claim 5, or a salt thereof, which is obtained by the preparation process according to claim 5, is subjected to esterification.

21. A process for the preparation of a compound of the formula: 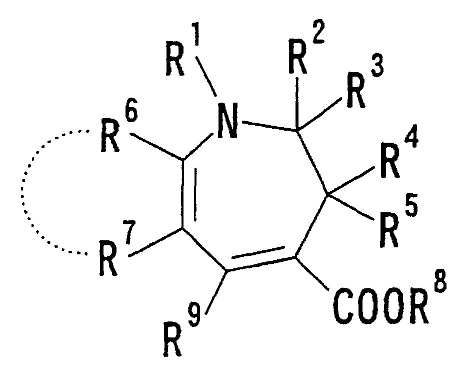 wherein R⁹ is a hydrogen atom or an optionally substituted hydrocarbon group, and the other variables are as defined below, or a salt thereof, **characterized in that** a compound of the formula: 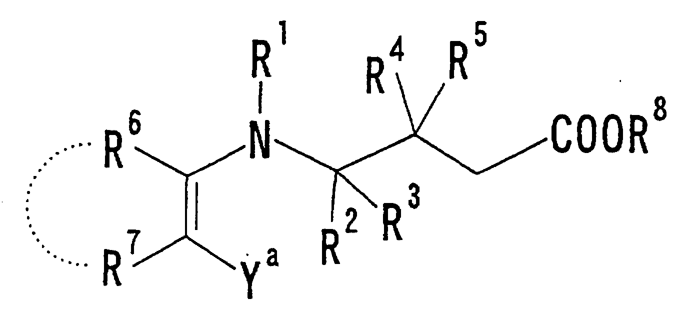 wherein Y^{a} is a group of formula -COR⁹ wherein R⁹ is a hydrogen atom or an optionally substituted hydrocarbon group, and the other variables are as defined in claim 16, or a salt thereof, which is obtained by the preparation process according to claim 16, is subjected to ring-closing reaction.

22. The preparation process according to claim 21, wherein R⁹ is a hydrogen atom.

23. The preparation process according to claim 21, wherein R², R³, R⁴, and R⁵ are hydrogen atoms.

24. The process according to claim 21, wherein R¹ is an optionally substituted hydrocarbon group.

25. The preparation process according to claim 21, **characterized in that** a compound of the formula: 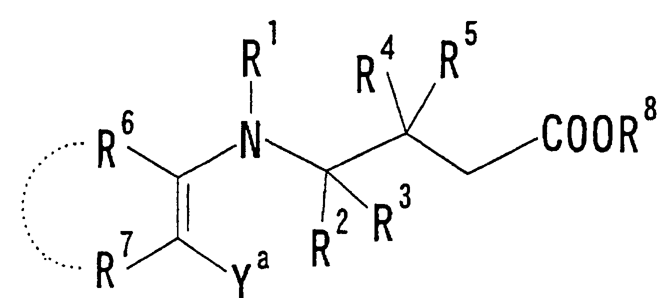 wherein Y^{a} is a group of formula -COR⁹ wherein R⁹ is a hydrogen atom or an optionally substituted hydrocarbon group, and the other variables are as defined in claim 16, or a salt thereof, which is obtained by the preparation process according to claim 16, is subjected, without being isolated, to ring-closing reaction.

26. A process for the preparation of a compound of the formula: 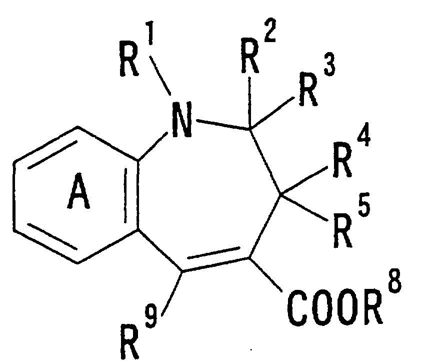 wherein R⁹ is a hydrogen atom or an optionally substituted hydrocarbon group, and the other variables are as defined below, or a salt thereof, **characterized in that** a compound of the formula: 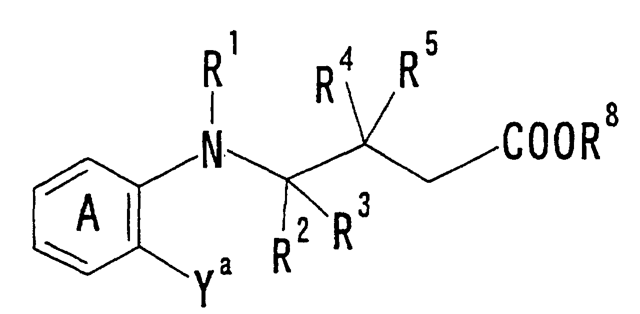 wherein Y^{a} is a group of formula -COR⁹ wherein R⁹ is a hydrogen atom or an optionally substituted hydrocarbon group, and the other variables are as defined in claim 20, or a salt thereof, which is obtained by the preparation process according to claim 20, is subjected to ring-closing reaction.

27. The preparation process according to claim 26, wherein R⁹ is a hydrogen atom.

28. The preparation process according to claim 26, wherein R², R³, R⁴, and R⁵ are hydrogen atoms.

29. The preparation process according to claim 26, wherein R¹ is an optionally substituted hydrocarbon group.

30. The preparation process according to claim 26, **characterized in that** a compound of the formula: 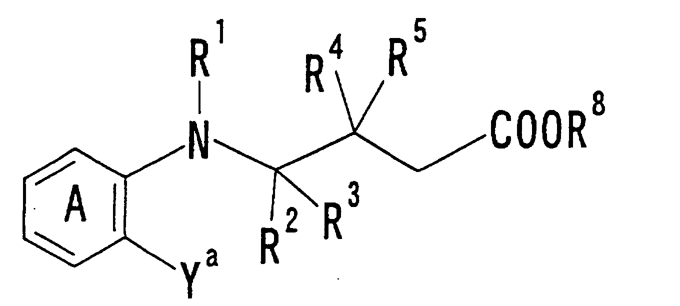 wherein Y^{a} is a group of formula -COR⁹ wherein R⁹ is a hydrogen atom or an optionally substituted hydrocarbon group, and the other variables are as defined in claim 20, or a salt thereof, which is obtained by the preparation process according to claim 20, is subjected, without being isolated, to ring-closing reaction.
